# EUROPEAN PATENT APPLICATION

(11) **EP 1 829 967 A1**
(43) Date of publication of application: **05.09.2007**
(21) Application number: 05811803.5
(22) Date of filing: 05.12.2005
(51) Int. Cl.: C12N 15/09, A61K 48/00, A61P 35/00, C12M 1/00, C12Q 1/68, G01N 33/543

(54) **METHOD OF DIAGNOSING MALIGNANT LYMPHOMA AND ESTIMATING THE PROGNOSIS THEREOF**

(30) Priority: 03.12.2004 US 632708 P; 30.09.2005 US 722007 P
(71) Applicant: Aichi Prefecture, Nagoya-shi, Aichi 460-0001 (JP); NGK INSULATORS, LTD., Nagoya-city, Aichi 467-8530 (JP)
(72) Inventor: SETO, Masao, Nagoya-city, Aichi, 465-0034 (JP); TAGAWA, Hiroyuki, Nabeyaueno Apt, Nagoya-city, Aichi, 464-0006 (JP); YOSHIDA, Yasuko, NGK INSULATORS, LTD., Nagoya-city, Aichi, 467-8530 (JP); KIRA, Shigeki, NGK INSULATORS, LTD., Nagoya-city, Aichi, 467-8530 (JP)
(74) Representative: Bufton, Karen A.J.
(86) International application number: PCT/JP2005/022316
(87) International publication number: WO 2006/059769

(57) **Abstract**

In order to more accurately analyze a change in the complicated gene copy number in malignant lymphoma and identify a region affected by an important genomic aberration in greater detail so that the results can be used in diagnosing the type of disease and performing prognosis, genome-wide array CGH is carried out and thus human chromosome 1 36.23 to p36.32, human chromosome 1 q42.2 to q43, human chromosome 2 p11.2, human chromosome 2 q13, human chromosome 17 p11.2 to p13.3, and human chromosome 19 p13.2 to p13.3 are identified.

## Description

### Technical Field

The present invention relates to methods for diagnosis and prognosis of malignant tumors, in particular, malignant lymphomas, and more particularly to methods for diagnosis and prognosis of mantle cell lymphoma.

### Background Art

Mantle cell lymphoma (MCL) is characterized by the translocation (11:14)(q13:q32) in the BCL1 gene, which results in overexpression of CCDN1, and is presumed to derive from naive pre-germinal center CD5⁺ cells (Seto et al., 1992; Jaffe et al., 2001). The identification of this translocation in virtually all cases of MCL with CCDN1 overexpression indicates that this gene translocation plays an important role in tumorigenic transformation (Jaffe et al., 2001). Despite the presence of such a common marker, experiments with transgenic mice overexpressing CCND1 have proved that this protein alone cannot induce lymphomas (Hinds et al., 1994; Lovec et al., 1994). Consequently, it is assumed that genomic alterations other than the 11q13 translocation are involved in the development and progression of MCL. To identify such additional alterations, several studies using CGH and chromosome banding analyses have been conducted (Monni et al., 1998; Bea et al., 1999; Cuneo et al., 1999; Bentz et al., 2000; Martinez-Climent et al., 2001; Bigoni et al., 2001; Allen et al., 2003). These studies show that genomic imbalances, such as genomic gain/amplification of 3q, 6p, 7p, 8q, 10p, 12q and 18q, and genomic loss/deletion of 1p, 6q, 8p, 9p, 11g, and 13q, frequently occur in MCL. Some genetic deregulations accompanying these genomic imbalances were also detected, such as deregulations of the BMI-1 gene from amplification of 10p12.2, the p16^{INK4a} gene from deletion of 9p21.3, and the ATM gene from deletion of 11q22.3 (Dreyling et al., 1997; Pinyol et al., 1997; Stilgenbauer et al., 1999; Schaffner et al., 2000; Bea et al., 2001; Rosenwald et al., 2003). However, the target genes of the amplification and deletion sites remain unknown. One of the reasons for this is the limited resolution of chromosomal banding analysis or conventional CGH, which can detect only DNA copy number aberrations greater than 10 to 20 megabases.

Recently, a chip-based CGH approach with high resolution and accuracy, known as array CGH, has been developed (Pinkel et al., 1998). The present inventors established unique array CGH using a glass slide on which 2,348 bacterial artificial chromosomes (BACs) and P-1-derived artificial chromosomes (PACs) were each spotted in duplicate with an average resolution of 1.3 Mb. In addition, this array CGH could identify a novel tumor-related gene, C13orf25, at 13q31.3 in B-cell lymphomas (Ota et al., 2004). These results indicate that quantitative measurements of DNA copy number changes made with the array CGH can identify more accurately regions of genomic imbalance and that this procedure could thus be a useful tool for identification of tumor-related gene(s).

### Disclosure of Invention

In order to analyze the complex gene copy number changes more accurately and to identify key gain/loss regions in greater detail, the present inventors performed genome-wide array CGH on 29 patient samples and 7 mantle cell lymphoma (MCL) cell strains.

In mantle cell lymphoma (MCL), the chromosome translocation of the BCL1 gene locus (11q13) plays an important role in tumorigenic transformation. It is also known that the chromosome translocation alone is not sufficient to cause tumorigenic transformation. However, genomic aberrations other than the translocation related to the type and pathologic conditions of MCL have not been studied in detail. Under these circumstances, with respect to MCL patient samples in 29 cases and 7 MCL cell strains, genomic aberrations have been examined over the whole-genome region using array CGH recently established by the present inventors. As a result, genomic aberrations characteristic to MCL have been found. Deletions in the BIM gene locus region have been revealed for the first time. As a result of search, deletions in the BIM gene locus region have been recognized in the patient specimens in 5 cases, and deletions have been recognized in 5 out of 7 cell strains. The common deletion region has been examined in detail, and it has been found that the responsible gene on the deletion region is the BIM gene. It has also been found that the expression is strongly suppressed in response to deletions of the gene. It is the first time in this report that BIM gene aberrations associated with malignant tumors have been found. Furthermore, the BIM gene is an antagonist to the BCL2 gene having an anti-apoptotic effect, and it is assumed that the deletions of the BIM gene enhance the anti-apoptotic function of the BCL2 gene in cells, and thus play an important role in tumorigenic transformation and the pathologic conditions thereof. In analyses conducted up to the present time, BIM gene aberrations have been recognized characteristically in MCL among malignant B-cell lymphomas.
Since BCL2 is expressed in many tumor cells, there is a possibility that the suppression of the expression of the BIM gene will also play an important role in tumorigenic transformation and formation of pathologic conditions in other solid cancers. That is, it is highly possible that the BIM gene will function as a cancer suppressor gene in tumorigenic transformation and formation of pathologic conditions, and the BIM gene can be an index of therapeutic response. There is also a possibility that the significance of the BIM gene as a target molecule will be clarified in the future.

According to the present invention, the following means are provided.
According to an embodiment of the present invention, there is provided a method for diagnosis of a malignant tumor including a step of detecting a deletion or mutation in one or two or more selected from the group consisting of human chromosome 1 p36.23 to p36.32, human chromosome 1 q42.2 to q43, human chromosome 2 p11.2, human chromosome 2 q13, human chromosome 17 p11.2 to p13.3, and human chromosome 19 p13.2 to p13.3.

In the method, the malignant tumor may be a malignant lymphoma. The method can further include a disease type determination step of determining the type of malignant lymphoma on the basis of the detection result in the detection step. Furthermore, in the disease type determination step, the disease type can be determined to be mantle cell lymphoma.

The method can further include a step of detecting a deletion or mutation in the base sequence of human chromosome 2 q13. The detection step can be a step of detecting a deletion of a region of human chromosome 2 q13 or a deletion of the BIM gene. The method can further include a therapeutic response determination step of determining a therapeutic response of the malignant tumor on the basis of the detection result in the detection step. That is, when a deletion is detected in a region of human chromosome 2 q13 or the BIM gene, the therapeutic response can be diagnosed as low.

Furthermore, in the method, the detection step can be a step of detecting a deletion or mutation in any of BAC RP11-438K19 35027bp to 49920bp, the 394th base to the 597th base in the base sequence described in Sequence ID No. 1, and the 214th base to the 417th base in the base sequence described in Sequence ID No. 2.

Furthermore, the detection step can be a step of using any of the BIM gene, and mRNA and cDNA expressed from the BIM gene, in a specimen. The detection step can include carrying out PCR, RT-PCR, or nucleic acid hybridization. Furthermore, the detection step can include a step of hybridizing, on an array provided with one or two or more probes complementary to at least a part of the BIM gene, the probes to a nucleic acid sample prepared from the specimen.

Furthermore, in the method, the detection step can be a step of performing array CGH.

Furthermore, in the method, the detection step can be a step of detecting the presence or absence of expression, an expression level, or a mutation of a protein encoded by the BIM gene.

According to another embodiment of the present invention, there is provided a diagnostic marker for malignant tumors, the diagnostic marker being the BIM gene, a part of the BIM gene, or a polynucleotide having a base sequence complementary thereto. In this description, the term "diagnostic marker" includes both a compound serving as an indicator of diagnosis and a compound that marks a compound serving as an indicator and is capable of detecting the compound. For example, the BIM gene can be considered as a compound serving as an indicator of diagnosis, and the polynucleotide having a base sequence complementary thereto can be considered as a compound capable of marking the BIM gene.

The marker can have a base sequence described in Sequence ID No. 1 or in Sequence ID No. 2, which is a protein translation region of the BIM gene. The marker may be at least a part of the 394th base to the 597th base in the base sequence described in Sequence ID No. 1 or the 214th base to the 417th base in the base sequence described in Sequence ID No. 2, or a polynucleotide having a base sequence complementary thereto.
Such a polynucleotide may be used as a probe or a primer.

According to another embodiment of the present invention, there is provided a diagnostic marker for malignant tumors, the diagnostic marker being a protein encoded by the BIM gene, a part thereof, or an antibody thereto.

According to another embodiment of the present invention, there is provided an array for diagnosis of malignant tumors, in which a nucleic acid probe for detecting a deletion or mutation in human chromosome 2 q13 is immobilized.

In the array, the probe can be a nucleic acid probe for detecting a deletion or mutation of the BIM gene. Furthermore, the nucleic acid probe may have at least a part of the 394th base to the 597th base in the base sequence described in Sequence ID No. 1 or the 214th base to the 417th base in the base sequence described in Sequence ID No. 2, or a base sequence complementary thereto.

According to another embodiment of the present invention, there is provided a diagnostic kit for any of the methods for diagnosis described above, the diagnostic kit including a nucleic acid probe for detecting a deletion or mutation of the BIM gene. The diagnostic kit may include a probe for detecting deletions in various regions in the chromosomes.

According to another embodiment of the present invention, there is provided a pharmaceutical composition for treating mantle cell lymphoma, containing a DNA construct including a coding region of the BIM gene or a coding region of a homologuous protein having an activity of a protein encoded by the BIM gene.

According to another embodiment of the present invention, there is provided a pharmaceutical composition for treating mantle cell lymphoma, containing a protein encoded by the BIM gene or a homologuous protein having an activity of a protein encoded by the BIM gene.

According to another embodiment of the present invention, there is provided a method for prognosis of a mantle cell lymphoma patient, the method including a step of detecting a deletion or mutation in human chromosome 6 q16.2 to q27, a deletion or mutation in human chromosome 8 p12 to p23.2, and an amplification or mutation in human chromosome 8 q13.2 to q24.22 with respect to a sample collected from the patient.

In the method, any one of the determination steps (a) to (c) described below can be carried out.
(a) When a deletion is detected in human chromosome 6 q16.2 to q27, the prognosis is good.
(b) When a deletion is detected in human chromosome 8 p12 to p23.2, the prognosis is poor.
(c) When an amplification is detected in human chromosome 8 q13.2 to q24.22, the prognosis is poor.

In the method, the detection step can be a step of detecting the deletion or amplification by hybridization of a probe containing a region on the chromosome to a nucleic acid sample collected from the patient. In this step, the probe may be a BAC clone and/or a PAC clone. Furthermore, in the item (a), a BAC clone (RP11-60019) can be used; in the item (b), a BAC clone (RP11-240A17) can be used; and in the item (c), a BAC clone (RP11-1136L8) or a PAC clone (RP1-80K22) can be used. Furthermore, the detection step can be carried out on a solid-phase carrier. In the method, the detection step can also be a step of performing array CGH.

In the method, the detection step can be a step of detecting an amplification, enhanced expression, or mutation of the c-MYC gene. The detection step may be a step of using any of the c-MYC gene, and mRNA and cDNA expressed from the c-MYC gene, in a specimen. The detection step can include carrying out PCR, RT-PCR, and nucleic acid hybridization. Furthermore, the detection step can include a step of hybridizing, on an array provided with one or two or more probes complementary to at least a part of the c-MYC gene, the probes to a nucleic acid sample prepared from the specimen.

Furthermore, in the method, the detection step can be a step of detecting the presence or absence, an expression level, or a mutation of a protein encoded by the c-MYC gene.

According to another embodiment of the present invention, there is provided an array for prognosis of mantle cell lymphoma, in which any of a probe for detecting human chromosome 6 q16.2 to q27, a probe for detecting human chromosome 8 p12 to p23.2, and a probe for detecting human chromosome 8 q13.2 to q24.22 is immobilized. The probe for detecting human chromosome 8 q13.2 to q24.22 may be a probe capable of detecting the c-MYC gene.

According to another embodiment of the present invention, there is provided a marker for prognosis of mantle cell lymphoma, the marker being a polynucleotide, such as a probe or a primer (set), for detecting human chromosome 6 q16.2 to q27, human chromosome 8 p12 to p23.2, and human chromosome 8 q13.2 to q24.22. The polynucleotide may be the c-MYC gene, a part of the c-MYC gene, or a polynucleotide having a base sequence complementary thereto.

According to another embodiment of the present invention, there is provided a marker for prognosis of mantle cell lymphoma, the marker being a protein encoded by the c-MYC gene, a part thereof, or an antibody thereto.

According to another embodiment of the present invention, there is provided a diagnostic kit including at least one of the markers for prognosis of mantle cell lymphoma described above.

According to another embodiment of the present invention, there is provided a pharmaceutical composition for treating mantle cell lymphoma, containing a nucleic acid construct that suppresses the expression of the c-MYC gene.

### Brief Description of the Drawings

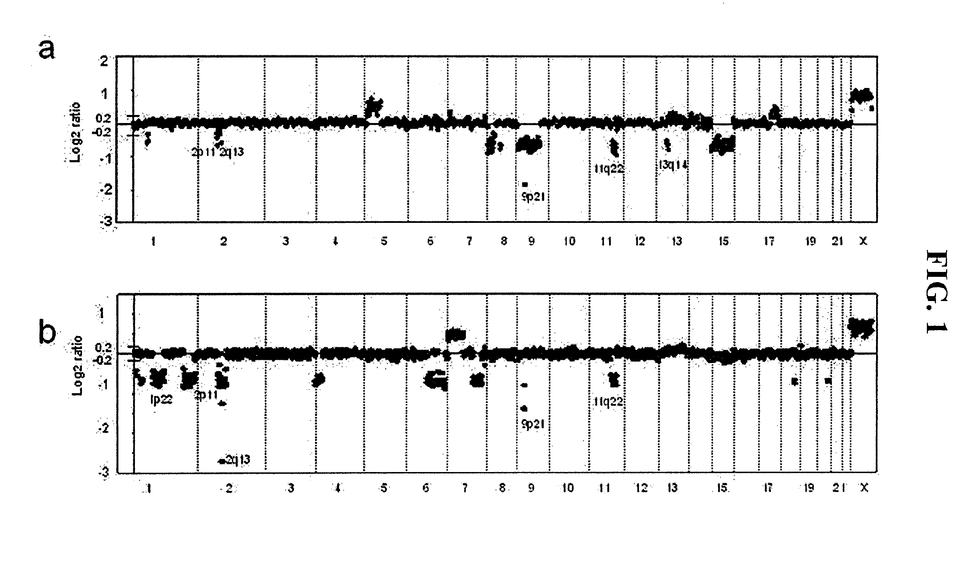

Fig. 1 shows representative genomic profiles for individual tumor patients. Whole genomic profiles are shown for a representative patient sample (a) and the SP-53 cell strain (b). Log₂ ratios are plotted for all clones on the basis of chromosome position with the vertical lines showing separation of chromosomes. The BACs and PACs are placed in order according to their position in the genome from the 1p telomere on the left to the Xq telomere on the right. (a) Regions of copy number gain: 5p, 7p21.3, 13q21.33, 17q21.31 to q24.3, and X. Regions of copy number loss: 1p32, 2p11.2, 2q13, 8p12 to p23.3, 8q12.3 to q13.1, 9p24.3 to q31.2, 11q22.3 to q23.2, 13q14.3 to q21.1, and 15. (b) Regions of copy number gain: 7p11.2 to p22.3. Regions of copy number loss: 1p36.23 to p36.32, 1p13.3 to p31.2, 1q13.2 to q44, 2p11.2 to q14.3, 4p15.1 to p16.1, 6q14.1 to q21, 6q23.2 to q26, 7q22.1 to q32.3, 9p21.3 to p22.1, 11q22.3 to q23.2, 18q22.1, and 20q13.13 to q13.2.

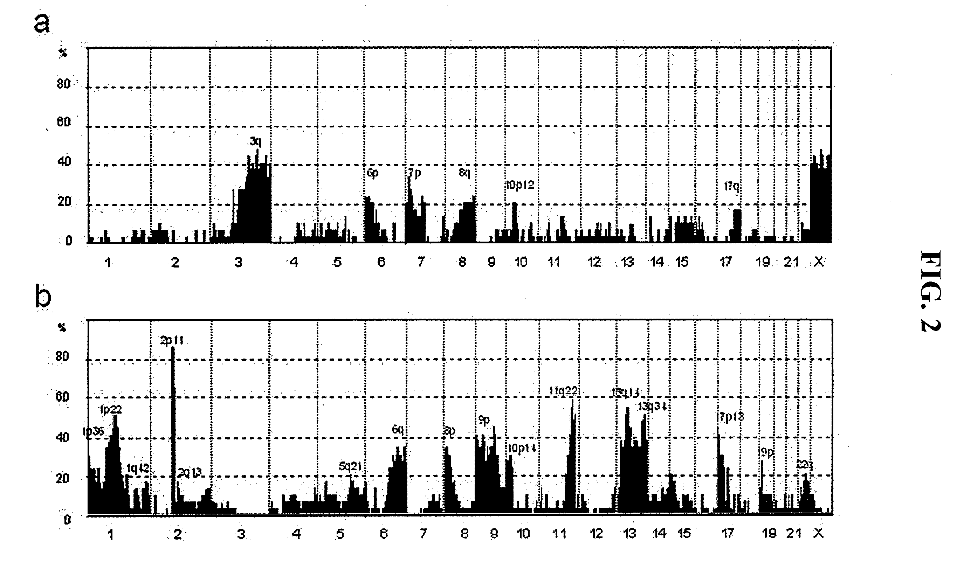

Fig. 2 shows genome-wide frequency of copy number alterations for 29 patients. (a) Frequency of copy number gains. (b) Frequency of copy number losses. Clones are placed in order from chromosome 1 to 22 and within each chromosome according to their Sanger Center mapping position, May 2004 version.

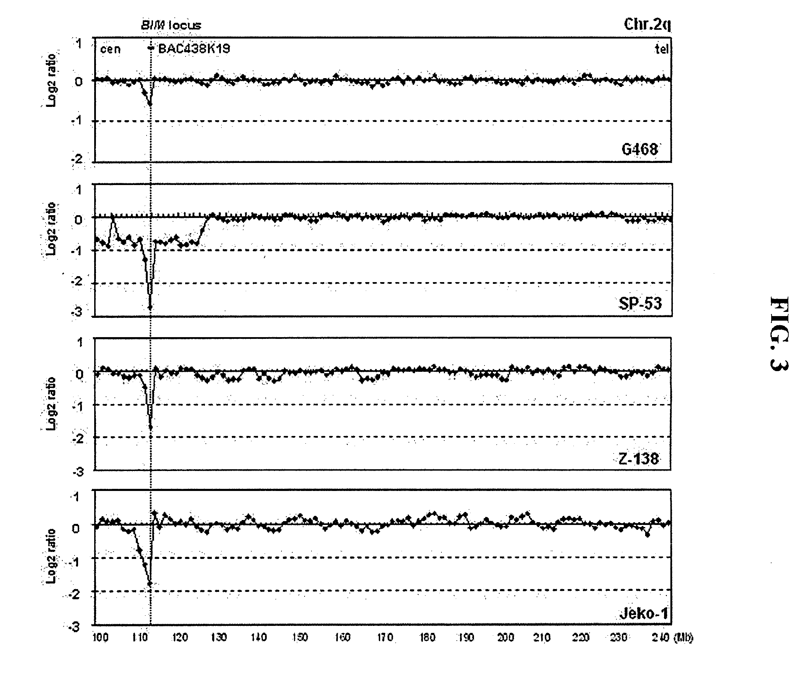

Fig. 3 shows genomic profiles of chromosome 2 from a patient sample (G468) and from three MCL cell strains (SP-53, Z-138 and Jeko-1). A log₂ ratio of +0.2 or more represents a genomic copy number gain, and a log₂ ratio of -0.2 or less represents a genomic copy number loss. Physical distances (Mb) from the 2q centromere are indicated. The vertical lines indicate the largest deletion of chromosome 2 at BAC438K19 containing the BIM gene. Log₂ ratios are -0.59 (G468), -2.75 (SP-53), -1.71 (Z-138), and -1.76 (Jeko-1) at BAC438K19, suggesting that homozygous loss occurs at the BIM gene locus.

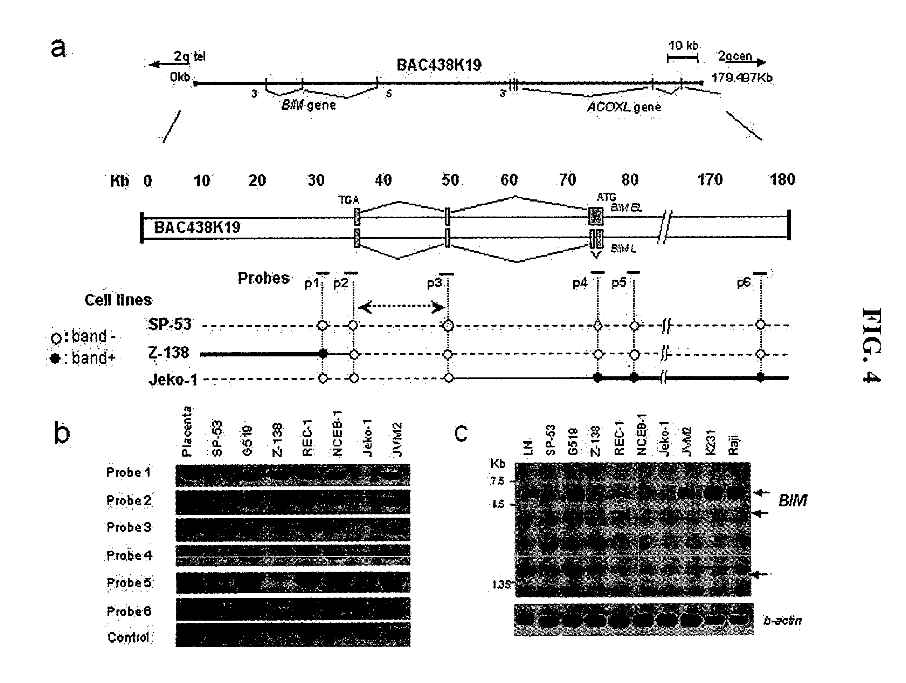

Fig. 4 shows the minimum common region of homozygous loss at 2q13 and expression of BIM. (a) Schematic illustration of BAC438K19, the BIM gene exons, and loss patterns of three cell strains (SP-53, Z-138, and Jeko-1). Gray boxes: exons (open reading frames of BIM EL and BIM L). The open reading frame of BIM EL (597 bp) consists of three exons: exon 1 from 75,082 to 75,475 bp (394 bp) including the initiating codon (ATG), exon 2 from 49,074 to 49,177 bp (104 bp), and exon 3 from 34,990 to 35,088 bp (99 bp) including the termination codon (TGA), all on BAC438K19. Black and white circles: probes used for Southern blot analyses. Broken horizontal lines with white circles: homozygous loss (bands negative). Thick horizontal lines with black circles: no homozygous loss (bands positive). Thin horizontal lines: presence or absence of homozygous loss is not confirmed. Bold broken horizontal arrows between probes 2 and 3 indicate the minimum common region of homozygous loss of 2q13. (b) Southern blot analyses using probes 1-6 for genomic DNAs of MCL cell strains. Lane 1, human placenta; lane 2, sp-53; lane 3, Granta 519 (G519); lane 4, Z-138; lane 5, REC-1; lane 6, NCEB-1; lane 7, Jeko-1; lane 8, JVM2. Bands of probe 1: human placenta (+), SP-53 (-), Granta 519 (+), Z-138 (+), REC-1 (+), NCEB-1 (+), Jeko-1 (-), and JVM2 (+). Bands of probe 4: human placenta (+), SP-53 (-), Granta 519 (+), Z-138 (-), REC-1 (+), NCEB-1 (+), Jeko-1 (+/-), and JVM2 (+). Bands of probes 5 and 6: human placenta (+), SP-53 (-), Granta 519 (+), Z-138 (-), REC-1 (+), NCEB-1 (+), Jeko-1 (+), and JVM2 (+). "Control" indicates the representative control band of probe 3 (TCR β probe) located under the bands of probe 6. (c) Northern blot analysis of BIM with seven MCL cell strains, and B-cell lymphoma (Karpas 231) and Burkitt's lymphoma (Raji) cell strains. Control is β-actin.

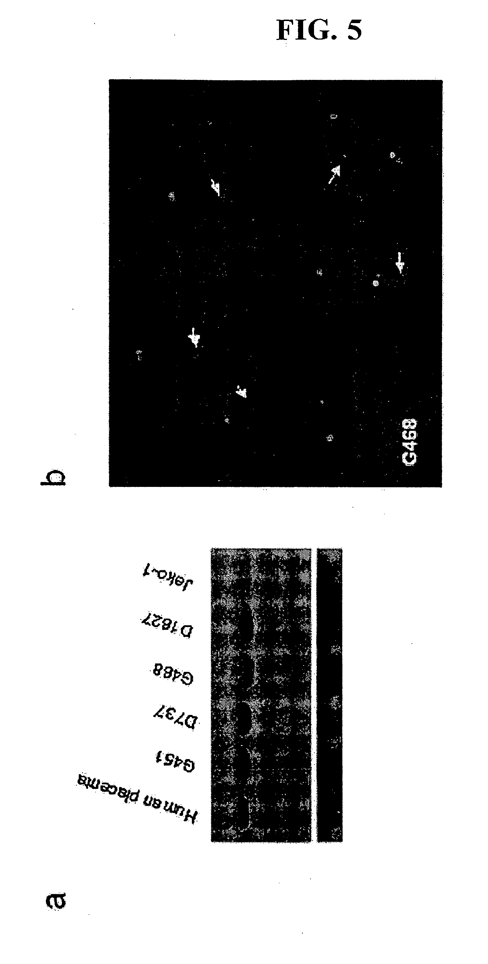

Fig. 5 shows results of Southern blot analysis and FISH analysis of a patient sample (G468). (a) Southern blot analysis. Lane 1: human placenta; lanes 2, 3, and 5: patient samples without 2q13 loss; lane 4: a patient sample G468 showing 2q13 loss by array CGH (see Fig. 3); lane 6: Jeko-1 cell strain showing homozygous loss at the BIM gene locus. Probe 2 containing the BIM exon was used in this experiment. (b) Dual-color FISH analysis with probes A and B of G468. Probe A: BAC438K19; probe B: BAC368A17. Probe B is 1.55 Mb telomeric to probe A, and BAC438K19 contains the BIM gene. Interphase chromosomes have two pairs of red signals (probe B, red), and one pair of green signals (probe A, green), indicating heterozygous loss of probe A.

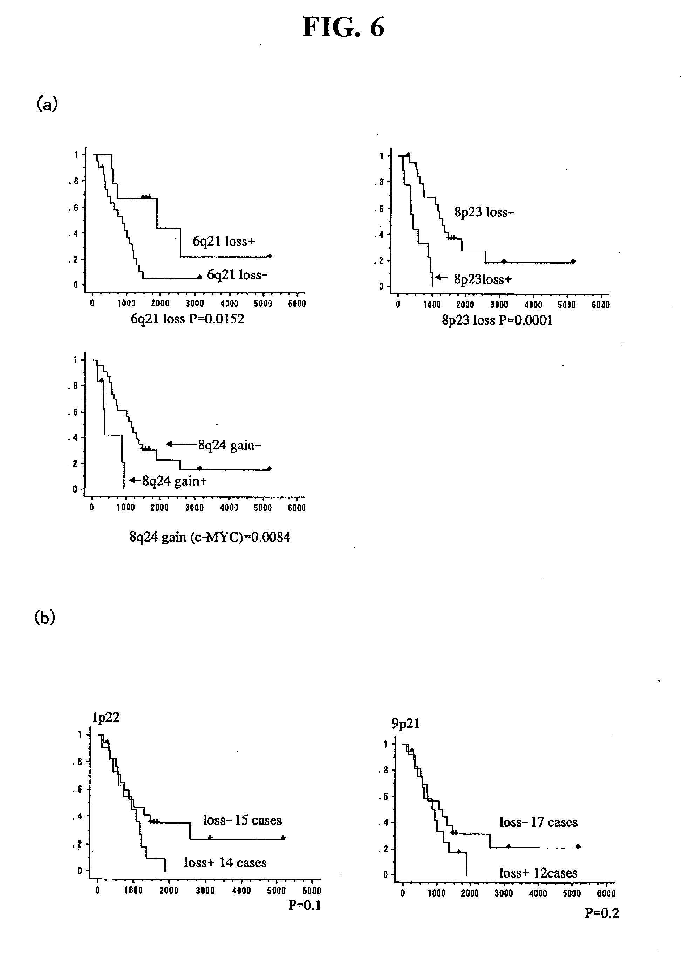

Fig. 6 (a) shows Kaplan-Meier survival curves for mantle cell lymphoma in the cases with and without loss in 6q21, with and without gain in 8p23, and with and without gain in 8q24. The abscissa represents the number of days of survival, and the ordinate represents the survival probability. Fig. 6 (b) shows Kaplan-Meier survival curves for mantle cell lymphoma in the cases with and without loss in 1p22 and with and without loss in 9p22.

### Best Mode for Carrying Out the Invention

### (Method for diagnosis of malignant tumor)

A method for diagnosis of a malignant tumor of the present invention is characterized by including a step of detecting a deletion or mutation in one or two or more selected from the group consisting of human chromosome 1 p36.23 to p36.32, human chromosome 1 q42.2 to q43, human chromosome 2 p11.2, human chromosome 2 q13, human chromosome 17 p11.2 to p13.3, and human chromosome 19 p13.2 to p13.3.

According to the diagnosis method, when a malignant tumor patient sample is subjected to such a detection step and when a homozygous or heterozygous deletion is found in the chromosome region described above, it is possible to make a diagnosis of a malignant tumor. Among the various regions on the chromosomes, it is useful to detect a deletion or mutation in human chromosome 2 q13. The deletion occurs with high frequency in this region in MCL case specimens and also in MCL cell strains. The mutation is characteristic in malignant lymphomas, in particular, malignant B-cell lymphomas. In general, MCL cell strains exhibit a higher grade of malignancy than MCL case specimens. Consequently, the detection of a deletion or mutation in 2q13 is useful for diagnosing the type of high-grade-malignancy lymphoma, such as MCL, among malignant lymphomas, in particular, among malignant B-cell lymphomas.

With respect to human chromosome 17 p11.2 to p13.3, it is preferable to detect a deletion or mutation in human chromosome 17 P13.3 and p13.1. With respect to human chromosome 19 p13.2 to p13.3, it is preferable to detect a deletion or mutation in human chromosome 19 p13.2. The reason for this is that these regions have a higher frequency of deletion or mutation.

In order to detect a deletion or mutation in the regions on the chromosomes, any of various methods may be employed. For example, the detection can be performed by hybridization using a nucleic acid sample collected from a patient and a probe that hybridizes to the regions on the chromosomes. A nucleic acid sample from a case or the like can be obtained from a lymphoma sample from a patient or the like using a standard DNA extraction method or the like. As the probe, any probe that hybridizes to at least a part of such a region on the chromosome may be used. As the DNA probe, a BAC clone and/or a PAC clone corresponding to the chromosome region can be used. Examples of the regions on the chromosomes and the clones are shown in Tables 1 and 2.

The form of hybridization is not particularly limited. The hybridization may be a liquid-phase reaction or a method using a solid-phase carrier, such as beads or a basal plate. It is preferable to use a DNA probe, such as a clone, immobilized on a solid-phase carrier, such as a chip. Examples of the solid-phase carrier on which a DNA probe is immobilized as described above include a DNA array. The immobilization form of the probe is not particularly limited, but includes immobilization by any of various bonds, such as covalent and/or noncovalent bonds, such as electrostatic bonds and hydrophobic interactions. Furthermore, as the array in which such a probe is immobilized, for example, an array used for array CGH can be used.

As described above, according to this embodiment, a solid-phase carrier on which such a probe is immobilized is also provided. Typically, the solid-phase carrier is a flat basal plate, such as a slide glass. Examples of a carrier for probe immobilization include a DNA microarray.

The present inventors have identified that the responsible gene for the deletion of 2q13 is the BIM gene. Consequently, in order to detect a deletion or mutation in 2q13, a deletion or mutation in the BIM gene may be detected. In such a case, the deletion or mutation can be detected by the deletion itself in the BIM gene in the specimen, and also by the expression level of the BIM gene. That is, when the expression level of the BIM gene in the specimen sample is low compared with the sample of a healthy subject, it is possible to diagnose the type of high-grade-malignancy lymphoma, such as MCL, among malignant lymphomas, in particular, among malignant B-cell lymphomas. In the case where the expression level of the BIM gene in the specimen sample is low compared with the sample of a healthy subject, for examle, the expression level is preferably 60% or less of that of the healthy subject, more preferably 50% or less, still more preferably 20% or less, and still more preferably 10% or less.

In the detection of a deletion or mutation in the BIM gene, specifically, any of the BIM gene, and mRNA and cDNA expressed from the BIM gene in a specimen can be used. Such a nucleic acid material can be obtained by a known method of collecting or amplifying a nucleic acid sample, such as PCR or RT-PCR. The primer used in PCR capable of amplifying the BIM gene or a part thereof can be designed on the basis of the sequence of the BIM gene. Preferably, the primer can be designed on the basis of a sequence described in Sequence ID No. 1 or Sequence ID No. 2, which is a protein translation region of the BIM gene, or the 394th base to the 597th base in the base sequence described in Sequence ID No. 1 or the 214th base to the 417th base in the base sequence described in Sequence ID No. 2. The base length of the primer is preferably 15 to 40 bases, and desirably 15 to 30 bases. However, when long accurate (LA) PCR is carried out, the base length is preferably at least 30 bases. It is preferable to select a base sequence so as to prevent annealing of sense and antisense strands and so that the formation of a hairpin structure can be avoided.

In order to detect a deletion or mutation in the BIM gene using such a nucleic acid sample, it is possible to carry out quantitative PCR, such as Real-Time PCR using such a primer (set).

Furthermore, in order to detect a deletion or mutation in the BIM gene using such a nucleic acid sample, it is possible to carry out quantitative PCR, such as Real-Time PCR using a suitable primer capable of amplifying the BIM gene.

Furthermore, in order to detect a deletion or mutation in the BIM gene, it is preferable to carry out hybridization using a probe that hybridizes specifically to the BIM gene. As such a probe, it is possible to use a probe that is complementary to at least a part of the BIM gene. The probe must hybridize specifically to a nucleic acid sample derived from the BIM gene, but it is not necessary that the probe is fully complementary to the gene. Such a polynucleotide having some alteration can be obtained by site-specific mutagenesis (Current Protocols in Molecular Biology, Ausubel et al., (1987) John Wiley & Sons, Chapters 8.1 to 8.5), PCR (Current Protocols in Molecular Biology, Ausubel et al., (1987) John Wiley & Sons, Chapters 6.1 to 6.4), ordinary hybridization (Current Protocols in Molecular Biology, Ausubel et al., (1987) John Wiley & Sons, Chapters 6.3 and 6.4), or the like. The probe hybridizes to a nucleic acid sample derived from the BIM gene under stringent conditions. Here, the term "stringent conditions" refers to conditions which permit specific bonding between the probe and the nucleic acid sample, the specific bonding being selective and detectable. The stringent conditions are defined by the salt concentration, organic solvent (e.g., formamide), temperature, and other known conditions. That is, the stringency is increased by decreasing the salt concentration, increasing the organic solvent concentration, or increasing the hybridization temperature. For example, the stringent salt concentration is usually about 750 mM or less of NaCl and about 75 mM or less of trisodium citrate, more preferably about 500 mM or less of NaCl and about 50 mM or less of trisodium citrate, and most preferably about 250 mM or less of NaCl and about 25 mM or less of trisodium citrate. The stringent organic solvent concentration is about 35% or more formamide, and most preferably about 50% or more formamide. The stringent temperature is about 30°C or higher, more preferably about 37°C or higher, and most preferably about 42°C or higher. Examples of the other conditions include the hybridization time, concentration of washing agent (e.g., SDS), presence or absence of carrier DNA. By combining these conditions, it is possible to set various stringency levels. Note that the hybridization conditions described above are merely exemplary and that hybridization conditions can be appropriately set by a person skilled in the art in consideration of the nucleotide sequence, concentration, and length of the probe, reaction time, reaction temperature, reagent concentration, and other conditions.

The probe may be labeled appropriately as necessary. Although labeling can be performed using a radioisotope (RI) method or a non-RI method, use of the non-RI method is preferred. Examples of the non-RI method include fluorescent labeling, biotin-labeling, and a chemiluminescence method. Fluorescent labeling is preferred.

In order to detect a deletion or mutation of the BIM gene, for example, analysis can be made using *in situ* hybridization, Northern blotting, dot blotting, a DNA array, or the like. The form of hybridization in such a method is not particularly limited. As described above, the hybridization may be a liquid-phase reaction or a method using a solid-phase carrier, such as beads or a basal plate. It is preferable to use a DNA probe immobilized on a solid-phase carrier, such as a chip or beads. Examples of the solid-phase carrier on which a DNA probe is immobilized include a DNA array (DNA chip). The immobilization form of the probe is not particularly limited, but includes immobilization by any of various bonds, such as covalent and/or noncovalent bonds, such as electrostatic bonds and hydrophobic interactions. The probe may be immobilized on a solid-phase carrier by *in situ* synthesis.

Furthermore, the detection of a deletion or mutation in 2q13 can be carried out by a step of detecting a deletion or mutation in any of BAC RP11-438K19 35027bp to 49920bp, the 394th base to the 597th base in the base sequence described in Sequence ID No. 1, and the 214th base to the 417th base in the base sequence described in Sequence ID No. 2. That is, these regions are DNA regions deleted in MCL cell strains in which a deletion of 2q13 and a decrease in the expression level of the BIM gene are recognized in Northern blotting (refer to Examples). The base sequences in the regions specified above in Sequence ID Nos. 1 and 2 are parts of the coding region of the BIM protein. Consequently, by detecting a deletion or mutation in these regions with any of the various methods described above using these base sequences or a part thereof as a probe or primer, it is possible to detect a deletion or mutation in 2q13, a deletion of the BIM gene, or the like.

On the basis of what is described above, according to the embodiment, there is provided diagnostic markers corresponding to the regions on various chromosomes, such as 2q13, the BIM gene, or a part thereof, or having a base sequence complementary thereto. By detecting a deletion or mutation in the markers, it is possible to diagnose the grade of malignancy and type of malignant tumor. Such a marker is preferably a nucleic acid molecule, such as polynucleotide, that can detect a deletion or mutation in the various regions or the BIM gene. Examples of the nucleic acid molecule include the regions on various chromosomes or a part thereof, polynucleotide having a base sequence complementary thereto, the BIM gene or a part thereof, and polynucleotide having a base sequence complementary thereto. The polynucleotide is preferably DNA. In particular, a nucleic acid molecule, such as any of the regions on various chromosomes or a part thereof, the BIM gene or a part thereof, or a probe or primer having a base sequence complementary thereto can be preferably used as a diagnostic reagent.

According to the embodiment, there is provided a probe or a DNA microarray on which a probe is immobilized. A diagnostic kit including such a primer set is also provided.

According to another embodiment of the method for diagnosis, the detection of a deletion or mutation in the BIM gene may be performed by detecting the presence or absence of the expression, an expression level, or an mutation of a protein encoded by the BIM gene. For that purpose, for example, an antibody specific for such a protein can be used. As the antibody, a polyclonal antibody or a monoclonal antibody can be used. An antobody molecule or a part thereof may be used. With respect to such an antibody, for example, in the case of a polyclonal antibody, an animal is immunized using a protein or a fragment thereof as an immunogen, and then the polyclonal antibody can be obtained from a serum. Alternatively, an expression vector for the eukaryotic cell is introduced into the muscle or skin of an animal by injection or using a gene gun, and then a serum is collected to obtain the polyclonal antibody. Examples of the animal to be used include mice, rats, rabbits, goats, and chickens. Furthermore, a monoclonal antibody can be produced according to a known monoclonal antibody production method (refer to, "Tankuron kotai (Monoclonal Antibody)" Takaaki Nagamune and Hiroshi Terada, Hirokawa Shoten, 1990; and "Monoclonal Antibody" James W. Goding, third edition, Academic Press, 1996).

The antibody may be labeled appropriately with a labeled substance. As the labeled substance, an enzyme, a radioisotope, or a fluorescent dye can be used. Examples of the enzyme include, but are not particularly limited to, enzymes used in ordinary EIA, such as peroxidase, β-galactosidase, alkaline phosphatase, glucose oxidase, acetylcholine esterase, glucose 6-phosphate dehydrogenase, and malate dehydrogenase. Furthermore, an enzyme inhibitor, a coenzyme, or the like can also be used. Binding of the enzyme to the antibody can be performed by a known method using a crosslinking agent, such as a maleimide compound. As a substrate, a known substance can be used according to the type of an enzyme to be used. As the fluorescent dye, fluorescent isothiocyanate (FITC), tetramethylrhodamine isothiocyanate (TRITC), or the like, which is used in the ordinary fluorescent antibody method, can be used.

In order to detect the expression level of the BIM protein or the like using such an antiboy, it is possible to use a measurement process including any of various detection methods, for example, immunostaining, such as staining of tissues or cells, competitive or noncompetitive radioimmunoassay (RIA), fluoroimmunoassay (FIA), luminescence immonoassay (LIA), and enzyme immunoassay or enzyme-linked immunosorbent assay (EIA or ELISA). In such a measurement process, the antigen-antibody reaction may be carried out either in a liquid phase or in a solid phase. In the detection, preferably, the antigen-antibody reaction product is separated. For that purpose, for example, chromatography or a solid-phase carrier, such as beads or a plate, may be used. A Western blotting technique may also be used. Furthermore, ELISA or the like may be used. It is also possible to use an array in which an antibody is immobilized on a solid-phase carrier, such as a basal plate.

According to the embodiment, there is provided a diagnostic marker for malignant tumors, containing antibody for the BIM gene or a part thereof. In particular, an antibody is preferable as a reagent used for detecting such a protein. According to the embodiment, there is also provided a diagnostic kit for malignant tumors containing such an antibody. In the diagnostic kit of the present invention, an antibody or a labeled antibody may be incorporated into a liquid phase. Alternatively, an antibody or a labeled antibody may be bound to a solid-phase carrier. The diagnostic kit may also contain an immobilized antigen or a part thereof. When the antibody is labeled with an enzyme, the diagnostic kit may contain a substrate thereof. Furthermore, when a solid-phase carrier is involved, the diagnostic kit may contain a washing agent for removing, by washing, molecules not bound to the solid phase. In addition, the diagnostic kit may contain any element that can be incorporated in a common diagnostic kit containing an antobody.

A pharmaceutical composition for treating MCL according to the present invention contains a DNA construct including a coding region of the BIM gene or a coding region of a homologuous protein having an activity of a protein encoded by the BIM gene. According to the present inventors, it has been found that the deletion of the BIM gene increases the grade of malignancy of malignant tumor. Consequently, in the MCL patient, it is expected that supplement of the BIM protein will suppress the progression of MCL. For that purpose, preferably, the DNA construct is introduced into the patient using a vector or the like to express the BIM gene. Therefore, by allowing a known vector to carry the coding region, the vector can be used as a pharmaceutical composition. Furthermore, by building a cell that carries the coding region in an expressible manner using such a vector or another transfection method, the cell can be used as a pharmaceutical composition.

According to another embodiment, a protein or the like encoded by the BIM gene can be directly used as a pharmaceutical composition.

The formulation and administration of such a pharmaceutical composition can be performed by a person skilled in the art by selecting an appropriate form and dosage. With respect to the pharmaceutical composition, for example, the DNA can be directly introduced using a virus vector, such as a retrovirus vector, an adenovirus vector, or an adeno-associated virus vector, or a liposome. The desired DNA or the like can be administered to the patient by an ex vivo method or *in vivo* method using any of the vectors or the like described above.

### (Method for prognosis of mantle cell lymphoma patient)

A method for prognosis of a mantle cell lymphoma patient is characterized by including a step of detecting any one of a deletion in human chromosome 6 q16.2 to q27, a deletion in human chromosome 8 p12 to p23.2, and an amplification in human chromosome 8 q13.2 to q24.22 with respect to a sample collected from the patient. In the method, any one of the determination steps (a) to (c) described below can be carried out.
(a) When a deletion is detected in human chromosome 6 q16.2 to q27, the prognosis is good.
(b) When a deletion is detected in human chromosome 8 p12 to p23.2, the prognosis is poor.
(c) When an amplification is detected in human chromosome 8 q13.2 to q24.22, the prognosis is poor.

Although each of these regions on the chromosomes is significantly related to the prognosis of MCL, from the standpoint of the frequency of mutation, with respect to human chromosome 6 q16.2 to q27, it is preferable to examine human chromosome 6 6q21 to q24.1 and more preferable to examine human chromosome 6q23.2 to q24.1. From the similar standpoint, with respect to human chromosome 8 p12 to p23.2, it is preferable to examine human chromosome 8 p23.1 to p23.2. Furthermore, with respect to human chromosome 8 q13.1 to q24.22, it is preferable to examine human chromosome 8 q21.3 to q24.21.

In terms of prognosis, the deletion in human chromosome 8 p12 to p23.2 is most significant. Next, the amplification in human chromosome 8 q13.1 to q24.22 and the absence of the deletion in human chromosome 6 q16.2 to q27 are significant in that order.

The method can include a step of detecting the deletion or amplification by hybridization of a probe containing the region on the chromosome to a nucleic acid sample collected from the patient. The probe may be a BAC clone and/or a PAC clone. Specifically, it is possible to use a BAC clone (RP11-60019), a BAC clone (RP11-240A17), and a BAC clone (RP11-1136L8) or a PAC clone (RP1-80K22).

In accordance with this embodiment, there is also provided a solid-phase carrier for immobilizing a probe for prognosis of the MCL patient, on which any of the probes described above is immobilized. A typical example is a DNA microarray. There is also provided a primer (set) capable of amplifying such a region on chromosome or a probe that hybridizes to such a region.

It is strongly suggested that the responsible gene on the amplification region in 8q24 is the c-MYC gene. Consequently, as in the BIM gene described above, it is also possible to carry out prognosis by detecting the expression level of the c-MYC gene. That is, when the expression level of this gene is high compared with that of a healthy subject, the prognosis can be considered poor. Specifically, the expression level is preferably 10% or more of that of the healthy subject, more preferably 30% or more, still more preferably 70% or more, and still more preferably 100% or more.

In order to detect the expression level of the c-MYC gene, for example, the same step as that of detecting the expression level of the BIM gene may be performed on the c-MYC gene or c-MYC protein. That is, the detection step can be a step of detecting an amplification, enhanced expression, or mutation of the c-MYC gene. The detection step can be a step of using any of the c-MYC gene, and mRNA and cDNA expressed from the c-MYC gene, in a specimen. Furthermore, the detection step can be a step of detecting the presence or absence, an expression level, or a mutation of a protein encoded by the c-MYC gene.

The present invention also covers an array, a marker for prognosis, such as a primer, a probe, or an antibody, and a prognosis kit, that can be used in the embodiments described above. That is, the array for prognosis of MCL may be provided with a probe capable of detecting the c-MYC gene. The marker for prognosis of MCL may be a probe or a primer (set), the marker being the c-MYC gene, a part of the c-MYC gene, or a polynucleotide having a base sequence complementary thereto. The marker for prognosis may be a protein encoded by the c-MYC gene, a part thereof, or an antibody thereto. Furthermore, there is provided a diagnostic kit including at least one of the markers for prognosis.

A pharmaceutical composition for treating MCL according to the present invention is characterized by containing a nucleic acid construct that suppresses the expression of the c-MYC gene. According to the present inventors, it has been found that the amplification of the c-MYC gene results in poor prognosis in MCL. Consequently, in the MCL patient, it is expected that suppression of the expression of the c-MYC protein in the MCL patient will suppress the progression of MCL. For that purpose, preferably, a nucleic acid construct capable of suppressing the expression of the c-MYC gene is introduced into the patient, directly or using a vector or the like, to suppress the expression of the c-MYC gene. As such a nucleic acid construct, it is possible to use a nucleic acid construct that can suppress the expression of the c-MYC gene by antisense or RNA interference. Examples of the nucleic acid construct include nucleic acid constructs prepared by an antigene nucleic acid method in which transcription is inhibited by an interaction, such as hybridization with DNA that encodes the gene or the like; an antisense nucleic acid method in which transcription or translation is inhibited by an interaction, such as hybridization with RNA, such as mRNA; an RNA interference method in which a transcript is split or translation is inhibited on the basis of an interaction, such as hybridization with a transcript, for example, mRNA; a decoy nucleic acid method; a ribozyme method; and the like. Furthermore, although the gene expression is not suppressed, an aptamer may be introduced by electroporation. Note that the term "nucleic acid" in the nucleic acid construct that suppresses the expression of the gene means a polynucleotide, such as deoxyribonucleic acid or ribonucleic acid. Furthermore, the term includes a single-stranded (DNA or RNA, sense or antisense) or double-stranded polynucleotide (DNA or RNA). The term also includes a DNA-RNA hybrid (double-stranded), a DNA-RNA chimeric oligonucleotide (single-stranded), peptide nucleic acid, and a morpholino oligonucleotide. Furthermore, the polynucleotide may be modified naturally or artificially.

For example, a nucleic acid construct capable of expressing RNA interference is constructed such that, using at least a part of a transcript, such as mRNA, of the target gene as a target, the expression of the target gene can be suppressed. One example of the nucleic acid construct is an RNA construct having a double-stranded structure including oligoribonucleotides that hybridize with each other. Specific examples thereof include relatively short double-stranded oligoribonucleotides, each with or without a protruding 3' end (small interfering RNA: siRNA) and a single oligoribonucleotide forming (or having) a hairpin structure (short hairpin RNA: shRNA). These RNA constructs are preferable from the standpoint that they can cause RNA interference directly. Furthermore, an RNA construct including a single-stranded oligoribonucleotide that does not form a hairpin structure can also express RNA interference.

Another example of the nucleic acid construct is a vector that encodes the RNA construct described above, i.e., siRNA or shRNA, in an expressible manner. Such a nucleic acid construct is preferable from the standpoint that RNA interference can be caused continuously. In the shRNA expression vector according to this example, an antisense sequence, a sense sequence, or a loop sequence can be constructed such that a continuous single-stranded RNA that can construct shRNA can be transcribed by intracellular transcription. The siRNA expression vector may be constructed such that RNA having a predetermined sense sequence and antisense sequence can be transcribed. In the siRNA expression vector, the sense sequence and antisense sequence may be expressed by a single vector or different vectors.

The formulation and administration of such a pharmaceutical composition can be performed by a person skilled in the art by selecting an appropriate form and dosage. With respect to the pharmaceutical composition, for example, the DNA can be directly introduced using a virus vector, such as a retrovirus vector, an adenovirus vector, or an adeno-associated virus vector, or a liposome. The desired DNA or the like can be administered to the patient by an ex vivo method or *in vivo* method using any of the vectors or the like described above.

While examples will be shown below to describe the implementability of the disclosed methods and the methods according to the claims, it is to be understood that the present invention is not limited to the examples. The scope of the following claims is to be accorded the broadest interpretation so as to encompass all modifications, equivalent structures and functions.

### EXAMPLES

### [Example 1]

### 1. Materials and Methods

### 1-1. MCL patients and samples

Tumor specimens obtained from 29 MCL cases, comprising 16 males and 13 females all from the Aichi Cancer Center, were included in the study. Twenty-four cases were classified as typical and five as blastoid variants. The median age of the patient was 67 years (49-92 years old). Eighteen out of 27 cases (67%) were leukemic (data of 27 cases were available), 27 out of 29 cases (93%) were in an advanced stage III-IV, eight out of 27 cases (30%) had elevated LDH (data of 27 cases were available), five out of 28 cases (18%) had a poor performance status (data of 28 cases were available), and 21 out of 27 cases (78%) had more than one extranodal site of involvement (data of 27 cases were available). The immunophenotype of the tumors was determined by immunohistochemistry for tissue sections and/or flow cytometry for cell suspensions. These studies used Ig light and heavy chains, several B-cell (CD19, CD20, CD22, CD45RA and CD79a) and T-cell (CD2, CD3, CD5, CD7, CD4, CD8, CD45RO and CD43) markers, CD10 and CD23. CCND1 expression was examined in all cases by Northern blot analysis and/or immunohistochemistry (Suzuki *et al.,* 1999). All tumors included in this study had a B-cell phenotype, co-expressed CD5, and showed CCND1 overexpression.

### 1-2. MCL cell strains

The seven MCL-derived cell strains used were SP-53, Granta 519, Z-138, REC-1, NCEB-1, Jeko-1 and JVM2. All these MCL cell strains have been thoroughly characterized in terms of morphology, immunophenotype, and/or interphase cytogenetics [detection of t(11;14)(q13;q32)] (Saltman et al., 1988; Amin et al., 2003; Jeon et al., 1998). The JVM2 cell strain, derived from a prolymphocytic leukemia and carrying t(11;14)(q13;q32), was also included in the study. Z-138, NCEB-1, Granta 519, REC-1 and JVM2 were kindly provided by Dr. Martin Dyer of Leicester University, UK. Karpas 231 derived from follicular lymphoma (FCL) and carrying t(14;18)(q32; q21) was kindly provided by Dr. Abraham Karpas of Medical Research Council Center, Hills Road, Cambridge, UK (Nachva et al., 1993). The Raji cell strain was derived from Burkitt's lymphoma.

### 1-3. DNA and RNA samples

High molecular weight DNA was extracted from 29 lymph nodes using standard Proteinase K/RNAse treatment and phenolchloroform extraction. Normal DNA was obtained from male and healthy blood donors. RNAs were prepared from cell strains by homogenization in guanidinium thiocyanate and centrifugation through cesium choride.

### 1-4. Array-based CGH

The array consisted of 2,348 BAC and PAC clones, covering the human genome at a resolution of roughly 1.3 Mb, from libraries RP11 and RP13 for BAC clones, and RP1, RP3, RP4 and RP5 for PAC clones. BAC and PAC clones were selected from the information in NCBI (http://www.ncbi.nlm.nih.gov/), Ensembl Genome Data Resources (http://www.ensembl.org/) and UCSC genome Bioinfomatics (http://www.ncbi.nlm.nih.gov/) and obtained from the BACPAC Resource Center at the Children's Hospital (Oakland Research Institute, Oakland, CA). Clones were ordered from chromosomes 1 to 22 and X within each chromosome on the basis of Ensembl Genome Data Resources from the Sanger Center Institute, February 2004 version. The locations of all the clones used for array CGH were confirmed by fluorescence in situ hybridization (FISH). Clone names and their chromosome locations are available on request.

The template for degenerate oligonucleotide-primed PCR (DOP-PCR) consisted of 10 ng of BAC (or PAC) DNA. DOP-PCR products were ethanol precipitated and dissolved in DNA spotting solution DSP0050 (Matsunami, Osaka, Japan) and robotically spotted in duplicate onto CodeLinkTM activated slides (Amersham Biosciences, Piscataway, NJ) using the inkjet technique by ceramic actuator (NGK, Nagoya, Japan).

Fabrication and validation of the array, hybridization methods and analytical procedures have been described elsewhere in detail (Ota et al., 2004). Briefly, one µg of tested (tumor or normal) and of referenced (normal) DNA was digested with DpnII and labeled with the BioPrime DNA labeling system (Invitrogen Life Technologies, Inc., Tokyo, Japan) using Cy3-dUTP and Cy5-dUTP (Amersham Pharmacia Biotech, Piscataway, NJ) for the tested and referenced DNA, respectively. Test and reference DNAs were then mixed with 100 µg of Cot-1 DNA (Life Technologies, Inc., Gaithersburg, MD), precipitated and resuspended in 45 µl of a hybridization solution (50% formamide, 10% dextran sulfate, 2x SCC, 4% SDS, and 100 µg tRNA) and hybridized onto a glass slide. After 48-66 hours' hybridization, the slide was washed and scanned with an Agilent Micro Array Scanner (Agilent Technologies, Palo Alto, CA) and the acquired array images were analyzed with Genepix Pro 4.1 (Axon Instruments, Inc., Foster City, CA). After automatic segmentation of the DNA spots and subtraction of the local background, intensities of the signals were determined. Subsequently, ratios of the signal intensity of two dyes (Cy3 intensity/Cy5 intensity) were calculated for each spot and converted into log₂ ratios on an Excel sheet in the order of chromosomal position.

For the array, six simultaneous hybridizations of normal male versus normal male were performed to define the normal variation for the log₂ ratio. A total of 113 clones with less than 10% of the mean fluorescence intensity of all the clones, with the most extreme average test over reference ratio deviations from 1.0 and with the largest SD in this set of normal controls were excluded from further analyses. Thus, we analyzed a total of 2,235 clones (covered 2,988 Mb, 1.3 Mb of resolution) for further analysis. 2,176 clones (covered 2,834 Mb) out of 2,235 were from chromosome 1p telomere to 22q telomere. 59 out of 2,235 clones were from chromosome X. Since more than 96% of the measured fluorescence log₂ ratio values of each spot (2 x 2,235 clones) ranged from +0.2 to -0.2, the thresholds for the log₂ ratio of gains and losses were set at the log₂ ratio of +0.2 and -0.2, respectively. Regions of low-level gain/amplification were defined as log₂ ratio +0.2 to +1.0, those suggested of containing a heterozygous loss/deletion as log₂ ratio -1.0 to -0.2, those showing high-level gain/amplification as log₂ ratio > +1.0, and those suggested of containing a homozygous losses/deletion as log₂ ratio < -1.0.

### 1-5. Southern blot analyses

To detect the target gene of 2q13 loss, probes 1-6 were designed from genomic DNA on BAC 438K19. The length of BAC 438K19 (Accession number: AC096670) is 179,497 bp. Probes 7 and 8 were designed from genomic DNA on BAC 368A17 (1.55 Mb telomeric to BAC438K19) and BAC537E18 (1.85 Mb centromeric to BAC 438K19) respectively. Probes 1-8 used by Southern blot analysis were amplified with the PCR method using eight primer pairs from human placenta DNA. The primer pairs used for PCR were as follows. Sequence ID Nos. 3 to 8 show the probes 1 to 6, and Sequence ID Nos. 9 to 24 show primer pairs for the probes 1 to 8.

Probe 1 (850 bp):
   sense (BAC438K19: 30,851-30,874 bp), 5'-ttgcacaagtaaagtggcaattac-3'
   antisense (BAC438K19: 31,700-31,677 bp), 5'-atccctgacaactcagcgtttaga-3'
Probe 2 (837 bp):
   sense (BAC438K19: 34,214-34,237 bp), 5'-acgaatggttatcttacgactgtt-3'
   antisense (BAC438K19: 35,050-35,027 bp), 5'-atctatgcatctgagtccagactg-3'
Probe 3 (850 bp):
   sense (BAC438K19 : 49,071-49,094 bp), 5'-taccctccttgcatagtaagcgtt-3'
   antisense (BAC438K19: 49,920-49,897 bp), 5'-tagtgacagcttaatgaaagggca-3'
Probe 4 (811 bp):
   sense (BAC438K19: 75,127-75,150 bp), 5'-gggtttgtgttgatttgtcacaac-3'
   antisense (BAC438K19: 75,937-75,914 bp), 5'-tgctgccctcagcattttcggcaa-3'
Probe 5 (1,095 bp):
   sense (BAC438K19: 80,501-80,524 bp), 5'-gggtttgtgttgatttgtcacaac-3'
   antisense (BAC438K19: 81,595-81,572 bp), 5'-ccgcgctggagttacaaactctat-3'
Probe 6 (890 bp):
   sense (BAC438K19: 177,361-177,384 bp), 5'-cattccccagaaacagatctcgtt-3'
   antisense (BAC438K19: 178,250-178,227 bp), 5'-catagcattatcaatgccatcgat-3'
Probe 7 (820 bp):
   sense (BAC368A17: 34,301-34,320 bp), 5'-ccatagttaatgtacacagc-3' antisense (BAC368A17: 35,101-35,120 bp), 5'-tcgcaaaccattaggaactg-3'
Probe 8 (500 bp):
   sense (BAC537E18: 191,071-191,094 bp), 5'-ttggagccaaggtaggattaaaca-3'
   antisense (BAC537E18: 191,487-191,570 bp), 5'-ctggaggaatagtgcttccagatg-3'

Probes 2-4 included an open reading frame of *BIM. BIM (BIM EL)* has several splice variants such as *BIM L, BIM* alpha, BIM beta and BIM gamma (O'Connor et al., 1998; U et al., 2001; Liu et al., 2002), and the open reading frame of *BIM EL* (597 bp) includes exons of these variants. Probe 4 includes the initiating codon (ATG) of *BIM (BIM EL* and *BIM* L), and Probe 2 the termination codon (TGA) of *BIM.*

Amplifications were performed on a Thermal Cycler (Perkin-Elmer Corporation, Norwalk, CT). PCR was conducted with the touchdown PCR method described elsewhere (Motegi et al., 2000). Briefly, the reactions consisted of 10 cycles of denaturation (94°C, 0.5 min), annealing (63° C, 0.5 min, 1° C decrease per 2 cycles), and extension (72°C, 2.5 min), followed by 25 cycles of denaturation (94°C, 0.5 min), annealing (58°C, 0.5 min), and extension (72°C, 2.5 min), and a final extension of 5 min at 72°C. The basic annealing temperature of the reaction ranged from 63°C to 58°C. All PCR products were separated by electrophoresis and purified with the QIA QuickTM Gel Extraction Kit (Qiagen). TA cloning to purified PCR products was performed with the aid of pBluescriptII SK (-), and sequenced with the ABI PRISMTM 310 Genetic Analyzer (Applied Biosystems, Foster City, VA). Ten µg of each genomic DNA sample was restriction digested for 16 hours with *Bam*H1 (for probes 1 and 6) or HindIII (for probes 2-4) and electrophoresed on a 0.8% agarose gel in 1x TBE. Gels were sequentially immersed in 0.25 M HCl for 30 min, 1.5 M NaCl/0.5 M NaOH for 30 min, and 0.5 M Tris (pH 7.4)/1.5 M NaCl for 30 min. Electrophoresed DNA was then transferred onto Hybond N+ membranes (Amersham Pharmacia Biotech, Tokyo, Japan), washed, and hybridized overnight at 65°C with [a-³²P]-dCTP-labeled probes 1-8. It was then washed, first with 2x SSC and then with diminishing concentrations of SSC-0.1% N-lauryl sarcosine at 65°C, and finally exposed to BioMaxTM MS films (EKC, Rochester, NY).

### 1-6. Northern blot analysis

Northern blotting was performed with *BIM EL* cDNA against seven MCL cell strains, Karpas 231 (FCL) and Raji (Burkitt's lymphoma). Probes used for Northern blot analysis were amplified with the RT-PCR method using the following primer pair (Sequence ID Nos. 25 and 26):
Sense: 5'-atggcaaagcaaccttctgatgta-3'
Antisense: 5'-tcaatgcattctccacaccaggcg-3'.

cDNA (open reading frame, 597 bp) of *BIM EL* was generated from fetal brain cDNA. Total cellular RNA (10 µg) was size-fractioned on a 1% agarose/0.66 M formaldehyde gel and transferred onto a Hybond-N+ nylon membrane (Amersham Pharmacia Biotech, Tokyo, Japan). The membranes were then hybridized overnight at 42oC with [a-³²P]-dCTP-labeled probes, washed, and finally exposed to BioMaxTM MS films.

### 1-7. Fluorescence in situ hybridization

Interphase chromosomes were prepared from paraffin embedded sample (G468) and cell strains. Dual color FISH analysis was conducted as previously described (Nomura et al., 2003; Zhang et al., 2004). Probes used in this experiment were probe A: BAC438K19 (green), and probe B: BAC368A17 (red).

### 2. Results

### 2-1. Genomic profiles of MCL patient samples and cell strains

Representative examples of the high-resolution analysis of a patient sample (G468) and SP-53 cell strain are shown in Figure 1a and 1b, respectively. Array CGH could detect both small and whole-chromosome areas of gains and deletions as well as delineate amplification and deletion borders. A small amplicon involving clones containing known oncogenes was easily detected, as were small homozygous deletions.

### 2-2. Genomic imbalances of MCL patient samples

Gains or losses of genetic material shown by all 29 patient samples were subjected to data analysis. The entire tumor set involved copy number gains on an average of 130.6 Mb or 4.6%, and copy number loss on an average of 250.6 Mb or 8.8% of the genome. Total alterations averaged 3.8 regions of gain and 7.3 regions of loss. The genome-wide frequency of copy number alterations, both gains and losses, are shown in Figure 2.

Regions of recurrent gain (> five cases) involved chromosomes 3q13.11-q29, 6p21.32-p25.3, 7p14.3-p22.3, 8q13.2-q24.22, 10p12.1-p12.2 and 17q23.2-q24.1, and recurrent losses (> five cases) localized at 1p36.23-p36.32, 1p11.2-p31.3, 1q42.2-q43, 2p11.2, 2q13, 5q21.1-q23.1, 6q16.2-q27, 8p12-p23, 9p24.3-q31.1, 10p12.31-p15.3, 11q14.3-q23.2, 13q13.2-q34, 15q13-q21.1, 17p11.2-p13.3, 19p13.2-p13.3 and 22q12.1-q13.1. The most frequent imbalances were gains of chromosomes 3q26.1 (48%), 7p21.1-p21.2 (34%), 6p25.3 (24%), 8q21.3-q24.21 (24%), 10p12.1-p12.2 (21%) and 17q23.2-q24.1 (17%) and losses of chromosome 2p11.2 (83%), 11q22.3-q23.1 (59%), 13q14.3-q21.1 (55%), 1p21.3-p22.1 (52%), 13q34 (52%), 9q22.33-q31.1 (45%), 17p13.3 (45%), 9p21.3-p22.1 (41%), 9p24.2-p24.3 (41%), 6q23.2-q24.1 (38%), 1p36.23-p36.32 (31%), 8p23.1-p23.3 (34%), 10p14-p15.3 (31%), 19p13.2 (28%), 5q22.1-q22.3 (21%), 22q12.2 (21%), 1q42.2-q43 (17%) and 2q13 (17%) (Table 1). Recurrent losses of 1p36.23-p36.32, 1q42.2-q43, 2p11.2, 2q13, 17p13.3 and 19p13.2-p13.3 were identified for the first time in this study, but no other regions of gains were found than those already listed in previous reports of studies using CGH for MCL.

**Table 1**

| | Recurrent regions^{a} | Most frequent regions^{b} | | | | |
|---|---|---|---|---|---|---|
| Gain/Loss | Chromosome^{c} | Chromosome | Mega base^{d} | No,of cases (%) | Clone^{e} | Gene^{f} |
| | 3q13.11-q29 | 3q26.1 | 162.6-170.1Mb | 13-14(45-48%) | RP11-576M8 | *SERPINI2* |
| | | (3q27.1) | 188.8Mb | 13(45%) | RP11-211G3 | *BCL6* |
| | 6p21.32-p25.3 | 6p22.3-p25.3 | 0.4-24.6Mb | 5-7(17-24%) | RP11-233K4 | *IRF4* |
| Gain | 7p14.3-p22.3 | 7p21.1-p21.3 | 8.7-19.8Mb | 9-10 (31-34%) | RP11-502P9 | *HS7c218* |
| | 8q13.2-q24.22 | 8q21.3-8q24.21 | 91.9-131.7Mb | 5-7(17-24%) | RP1-80K22 | MYC |
| | 10p12.1-p12.2 | 10p12.1-p122 | 22.9-25.8Mb | 5-6(17-21%) | RP11-301N24 | *BMI-1* |
| | 17q23.2-q24.1 | 17q23.2-q24.1 | 58.9-66.1Mb | 5(17%) | RP11-51F16 | - |
| | 1p36.23-p36.32 | 1p3623-p36.32 | 2.5-7Mb | 8-9(28-31%) | RP1-37J18 | - |
| | 1p11.2-p31.3 | 1p21.3-p22.1 | 95.4-101.2Mb | 14-15(48-52%) | RP4-561L24 | *GCLM* |
| | 1q42.2-q43 | 1q42.2-q43 | 230.9-238.2Mb | 5(17%) | RP11-781K5 | *Q8WUH8* |
| | 2p11.2 | 2p11.2 | 89.4-89.9Mb | 19-24 (66-83%) | RP11-136K15 | *KVIS(Igk)* |
| | 2q13 | 2q13 | 108.7-111.9Mb | 5(17%) | PP11-438K19 | *BIM* |
| | 5q21.1-q23.1 | 5q22.1-q22.3 | 104.8-114.5Mb | 5-6 (17-21%) | RP11-454E20 | - |
| | 6q16.2-q27 | 6q23.2-q24.1 | 133.3-146.5Mb | 10-11 (34-38%) | RP11-35612 | *TNFAIP3* |
| | 8p12-p23 | 8p23.1-p23.3 | 0.4-8.6Mb | 9-10(31-34%) | RP11-240A17 | - |
| Loss | 9p24.3-q31.1 | 9p24.2-p24.3 | 0.7-5.9Mb | 11-12 (39-41%) | RP11-130C19 | - |
| | | 9p21.3-p22.1 | 21.6-24Mb | 11-12 (39-41%) | RP11-14912 | *INK4α*/*ARF* |
| | | 9q22.33-q31.1 | 92.5-95.5Mb | 12-13 (41-45%) | RP11-54015 | *C9orf3* |
| | 10p12-31-p15.3 | 10p14-p15.3 | 2.2-18.7Mb | 8-9(28-31%) | RP11-401F24 | *C10orf47* |
| | 11q14.3-q23.2 | 11q22.3-q23.1 | 105-116.3Mb | 15-17(52-59%) | RP11-758F15 | *FDX* |
| | | (11q22.3) | 105-116.3Mb | 16(55%) | RP11-241D13 | *ATM* |
| | 13q13.2-q34 | 13q14.3-q21.1 | 46-50.8Mb | 15-16 (52-55%) | RP11-364119 | *RFP2* |
| | | 13q34 | 99-112.5Mb | 13-15 (45-52%) | RP11-65D24 | *bA65D24.2* |
| | 15q13-q21.1 | 15q13.1 | 22.4-41Mb | 5-6(17-21%) | RP11-125E1 | |
| | 17p11.2-p13.3 | 17p13.3 | 0.8-6.6Mb | 13(45%) | RP11-676J12 | *NXN* |
| | | 17p13.1 | 6.6-18.8Mb | 8-9 (28-31%) | RP11-199F11 | *TP53* |
| | 19p13.2-p13.3 | 19p13.2 | 6.4-7.9Mb | 7-9 (24-28%) | RP11-42J18 | *CD202* |
| | 22q12.1-q13.1 | 22q12.2 | 24.9-36.5Mb | 5-6(17-21%) | RPI-76B20 | *UCRX* |

Table 1 shows recurrent and most frequent regions of genomic gain and loss. Region of gain or loss was defined as the contiguity of at least three clones showing gain or loss, or, if not contiguous, clones showing a high copy number gain (log₂ ratio > +1.0) or a homozygous loss (log₂ ratio < -1.0). a: Recurrent region is defined as a region seen in > 5 of cases. b: The most frequent region of gain/loss was defined as the region with the highest frequency within each recurrent region. c: Regions are ordered according to their chromosomal position. d: According to Sanger Center Institute, February 2004 version. e: Representative of the most frequently gained or lost clone in each of the most frequent region. When the most frequently gained or lost clones share the same percentage of genomic aberrations in the most frequent regions, clones that include tumor related genes are shown above those do not. f: Genes contained in the representative clone.

Recurrent regions of high-level gains (log₂ ratio > +1.0) were found at 10p12.2 (two cases, BMI-1 gene locus), and recurrent regions of homozygous loss (log₂ ratio < -1.0) at 2p11.2 (three cases, *Ig_{K}* gene locus) and 9p21.1-p24.1. As shown in Table 2, the most frequently homozygously lost clone at 9p21-p24 was RP11-14912 (five cases), which contains the *p16^{INK4a}* tumor suppressor gene.

**Table 2**

| BAC name' | Genes contained Clones | Cytogenetic Position | Homozygous loss No. of Patients (n=29) | Homozygous loss No. of Cell line (n=7) |
|---|---|---|---|---|
| RP11-136K15 | *KVIS(Igk)* | 2p11.2 | 3^{b}(24)^{d} | 1^{c}(6)^{d} |
| RP11-438K19 | *BIM* | 2q13 | 0(5) | 3(5) |
| RP11-77E14 | *Q96GE9* | 9p24.1 | 0 (10) | 2 (4) |
| RP11-60C15 | *PTPRD* | 9p23-24.1 | 0(10) | 2(4) |
| RP11-380P16 | I*FNAS* | 9p22.1 | 2(11) | 1 (5) |
| RP11-14912 | *INK4α*/*ARF* | 9p21.3 | 5 (12) | 3(6) |
| RP11-214L15 | - | 9p21.3 | 2(10) | 0 (4) |
| RP11-393P6 | - | 9p21.3 | 2(11) | 1(4) |
| RPII-337A23 | *TEK* | 9p21.2 | 2(10) | 0(2) |
| RPII-205M20 | *TAFIL* | 9p21.1 | 2(7) | 0(1) |

Table 2 shows a list of BAC clones showing homozygous loss (log₂ ratio < -1.0). a: Clones are ordered according their chromosomal position. b: Number of patients showing homozygous loss in > 2 cases. c: Number of cell strains showing homozygous loss. d: Number of cases showing homozygous and heterozygous losses (log₂ ratio < -0.2).

All the 59 clones on chromosome X were analyzed separately because of sex mismatching, but the genomic alterations of X chromosomes were analyzed only for the 17 male patients. Two cases showed low-grade copy number gains of Xq28, while three cases showed heterozygous (two cases) or homozygous (one case) loss of Xp21.3-p22.3 with the most frequent region at Xp22.31-p22.32.

### 2-3. Genomic imbalances of MCL cell strains

Genomic imbalances generally occurred more frequently in MCL cell strains were than in those of patients. For example, gains of 7p21 and 8q24 (both n=3, 43%), and losses of 9p21 and 11q22 (both n=6, 86%), 1p22 (n=5, 71%) and of 6q, 8p23 and 13q21 (all n=3, 43%) in were more frequently detected in MCL cell strains than in patient samples.

Recurrent regions of high-level gain were detected at 13q31.3 (n=2, *C13orf25* gene locus) and at 18q21 (n=2, *BCL2* gene locus).Recurrent regions of homozygous loss were detected at 9p21.1-p24.1, 2p11.1 and 2q13 as seen in Table 2, which lists the homozygously lost clones of either patient samples or cell strains. Three cell strains (SP-53, Z-138 and Jeko-1) showed homozygous loss of 2q13 (log₂ ratio < -1.0), while two (REC-1 and NCEB-1) showed heterozygous loss at 2q13 (-1 < log₂ ratio < -0.2). Five patient samples with 2q13 deletion also displayed a heterozygous loss pattern. Individual partial genomic profiles of a patient sample (G468), and of cell strains SP-53, Z-138 and Jeko-1 of chromosome 2 are shown in Figure 3, which clearly indicates that the lowest locus of loss of 2q was at BAC, RP11-438K19 (BAC438K19), which contains two genes, BIM and ACOXL (Acyl-CoA dehydrogenase gene). The former is a BH3-only Bcl-2 family member protein that promotes apoptosis (O'Conner et al., 1998) while the function of the latter remains unknown. Because no information has been published regarding target gene(s) of homozygous loss at 2q13 , we next searched for the minimum common region of loss of 2q13 to help us detect candidate target gene(s).

### 2-4. Southern blot analyses

To detect the target gene of 2q13 loss, we performed Southern blot analyses of seven MCL cell strains using six genomic probes, which were designed from the genomic DNA of BAC438K19 (Figure 4a). The analyses using probes 1-6 demonstrated that partial exons of BIM were commonly deleted in the three cell strains SP-53, Z-138 and Jeko-1, and that the 'minimum common region' of homozygous loss is the *BIM* but not the *ACOXL* gene locus (Figure 4b). Here, 'minimum common region' represents the portion of the region that is aberrant in MCL cell strains with aberrations in a region. The minimum common region of homozygous loss of 2q13 of these three cell strains ranges at least from probes 2 to 3 (15 kb) and at most from probes 1 to 4 (45 kb).

This region includes the open reading frame of *BIM* but no other gene according to the NIBC, Ensembl Genome Data Resources and UCSC Genome Bioinfomatics. Southern blot analyses were also performed with probes from BAC, RP11-368A17 (probe 7) and BAC, RP11-537E18 (probe 8) for seven MCL cell strains. BAC, RP11-368A17 (BAC368A17) is a clone with a 1.55 Mb telomeric to BAC438K19, and BAC, RP11-537E18 (BAC537E18) a clone with a 1.85 Mb centromeric to BAC438K10. Bands of probes 7 and 8 were positive in all seven MCL cell strains (data not shown), indicating that region of homozygous deletion of each cell strain (SP-53, Z-138 and Jeko-1) is at a maximum 3.4 Mb. Furthermore, we performed Southern blot analysis of patient samples for which materials were available (Figure 5a), and found a heterozygous deletion pattern in a patient sample (G468) that showed heterozygous deletion at 2q13.

### 2-5. Northern blot analysis

To examine the expression of *BIM* in MCL cell strains, Northern blot analysis was performed of seven MCL cell strains, one FCL cell strain (Karpas 231) and one Burkitt's cell strain (Raji). As shown in Figure 4c, three transcripts of BIM, one major (5.7 kb) and two minor (3.8 kb and 1.35 kb) bands, were observed in Granta 519, JVM2, Karpas 231 and Raji whereas no or very weak expression was detected in SP-53, Z-138, Jeko-1, REC-1 and NCEB-1. Although array CGH data showed a heterozygous pattern at 2q13 in REC1 and NCEB1 cell strains, Northern blot analysis indicated that BIM mRNA in these two cell strains was clearly down regulated, which well be the result of a gene dosage effect. *BIM* was normally expressed at Granta 519 and JVM2, which showed no genomic loss at 2q13.

### 2-6. Fluorescence in situ hybridization

Dual color FISH using a combination of BAC438K19 and BAC368A17 (1.55 Mb telomeric to BAC438K19) and one of BAC438K19 and BAC537E18 (1.85 Mb centromeric to BAC 438K10) was performed on three MCL cell strains (SP-53, Z-138 and Jeko-1) and a patient sample (G468). These three clones were placed contiguously on our array CGH glass slide. Results of dual color FISH analysis using BAC438K19 and BAC368A17 for the patient sample (G468) are shown in Figure 5b. FISH results for these cell strains (data not shown) correlated well with the array CGH data. i) In the SP-53 cell strain, no signal of BAC438K19 was found whereas two pairs of BAC368A17 signals, or one pair of BAC537E18 signals was observed, indicating homozygous deletion of the BAC438K19 clone (log₂ ratio= -2.74). ii) In the Z-138 cell strain, one pair of a weak BAC438K19 signals was detected but two pairs of normal BAC368A17 signals or one pair of normal BAC537E18 signals was observed, suggesting intra-BAC438K19 deletion in this cell strain (log₂ ratio= -1.71). iii) In the Jeko-1 cell strain, one pair of weak BAC438K19 signals but two pairs of normal BAC368A17 signals, or one pair of weak BAC537E18 signals was observed, suggesting the deletion of intra-BAC438K19 in this cell strains (log₂ ratio= -1.76). These observations are concordant with the finding of total BAC438K19 deletion in SP-53 and partial BAC438K19 homozygous deletion in the Z-138 and Jeko-1 cell strains.

### 3. Discussion

In the study reported here, high-resolution mapping of copy number changes was achieved for the entire MCL genome. Frequent gains and losses could be identified with high resolution by means of array analysis, which allows for precise mapping of genomic aberrations. Although numerous genomic changes of MCL were identified by array CGH, many of them were the same as those previously listed in reports of studies using chromosomal CGH (also known as conventional CGH). Several authors (Monni et al., 1998; Beà et al., 1999; Bentz et al., 2000; Martinez-Climent et al., 2001; Allen et al., 2003) reported recurrent regions of gain as 3q (40-70%), 6p (20%), 7p (27%), 8q (20-30%), 10p (20%), 12q (20-30%), 18q21 (20%) and recurrent regions of loss as 1p (24-33%), 6q (27-37%), 8p (20-30%), 9p (16-30%), 11q (22-30%) and 13q (40-60%). However, the incidence of genomic aberrations identified by array CGH was generally higher than that reported in chromosomal CGH studies. For example, our data show more frequent losses of 1p21-p22 (52%) and 9p21 (41%, *INK4*/*ARF* locus) as well as of 11q22 (55%, *ATM* locus), than the corresponding losses previously detected by chromosomal CGH. Among these frequent losses, although the candidate target gene of 1p22 loss could not be identified, our array CGH analysis showed a most frequent region of loss within the 5.8 Mb region of 1p21.3-p22.1.

Recently, Kohlhammer and co-workers reported the results of their study of 49 patients for which they used array-based (matrix) CGH with glass slides on which 812 artificial chromosomes were spotted (Kohlhammer et al., 2004), and found higher frequencies of genomic alterations of MCL than those seen in chromosomal CGH data. Their patient characteristics (e.g. percentage of stage III/IV, poor performance status, high LDH level, leukemic MCL, and extra-nodal involvement) were almost the same as those of our series, as were the frequencies of genomic alterations. However, they could not identify several regions of loss detected by us, such as 1p36, 1q42.2-q43, 2p11.2, 2q13, 17p13.3 and 19p13.2-p13.3, because their clones were not selected from throughout the genome. The superior resolution of our study can thus be attributed to the unbiased selection of artificial chromosome clones from throughout the genome.

Of the six novel genomic regions of loss detected in our study, loss of 17p13.3 deserves special comment because it is highly interesting is that our array CGH analysis of 17p showed the most frequently deleted region(s) at 17p13.3, which suggests the existence of an additional tumor suppressor gene(s) distal to the *SP53* gene. Frequent allelic loss at 17p13.3 independent of the *SP53* locus has also been found in a variety of other human malignancies including lung, breast, ovarian and hepatocellular carcinomas as well as neural tumors (Fujimori et al., 1991; Cogen et al., 1992; Saxena et al., 1992; Phillips et al., 1996; Schults et al., 1996; Konishi et al., 2002). Although *SP53* mutation in MCL is a well-known genomic alteration and is associated with variant cytology and poor prognosis (Greiner et al., 1996; Hernandez et al., 1996), our finding indicated that other candidate tumor suppressor gene(s) at 17p13.3 may also be involved in the lymphomagenesis of MCL.

The key biological value of high-resolution array CGH lies in its ability to detect small, high-level gains in copy numbers and homozygous deletions that are capable of harboring specific oncogenes and tumor suppressor genes. Recurrent regions of high-level copy number gains have been identified as 1Op12.2 *(BMI-1),* 13q31.3 *(C13orf25)* and 18q21 *(BCL2)* (Beà et al., 2001; Ota et al., 2004; Hofmann et al., 2001; Martinez et al., 2003). Since biallelic (homozygous) loss is considered to be a hallmark of chromosomal regions harboring tumor suppressor genes (Kundson 1971), the detection of recurrent regions of homozygous loss at 2p11, 2q13 and 9p21-p24 is significant. Loss of 2p11 may be due to immunoglobulin gene rearrangement, but the loss region of 9p21-p24 covers nearly 15 Mb, making it difficult to identify the responsible gene(s) even though this region features the most frequently and homozygously deleted clone, RP11-149I2, which contains the *p16^{INK4a}* gene, which may well be the candidate gene for this region of homozygous loss of MCL (Dreyling et al., 1997; Pinyol et al., 1997).

While no previous studies have reported any candidate target gene of 2q13 among the three homozygous loss regions, our study showed that the minimum common region of 2q13 loss contains partial exons of *BIM* but no other genes or ESTs. This suggests that *BIM* appears to be the most likely target of this region of loss. It has recently become known that disturbances of pathways associated with apoptosis also contribute to the development of MCL (Hofmann et al., 2001; Martinez et al., 2003). Another study found that *BIM* is a pro-apoptotic *BCL2* family member and a major physiological antagonist of BCL2, particularly in hematopoietic systems (Bouillet et al., 2002), and Enders et al recently reported that B lymphocytes lacking Bim are refractory to apoptosis induced by B cell receptor ligation in vitro (Enders et al., 2003). Finally, Egle et al, using *Bim*^{*-*/}*⁻* and *Bim*^{*+*/}*⁻* Eµ-*Myc* mice, demonstrated that the loss of *Bim* was related to the onset of oncogenesis (Egle et al., 2004). These findings strongly suggest that *BIM* could be a tumor suppressor gene.

We demonstrated that the minimum common region of loss of 2q13 in MCL cell strains occurred at the *BIM* locus. Furthermore, we confirmed that the *BIM* expression of five out of seven MCL cell strains was down regulated while normal expression was found in two MCL cell strains without deletion of 2q13. These results constitute a powerful indication that *BIM* is the most likely candidate target gene of 2q13 loss/deletion and that its down regulation may contribute to tumorigenesis of MCL. In summary, the use of high-resolution array CGH technology for a detailed study of MCL allowed for an accurate identification of genomic aberrations and identification of *BIM* as a possible novel candidate tumor suppressor in MCL.

In summary, by using high-resolution array CGH for detailed studies of MCL, it is possible to accurately identify genomic aberrations and also to identify BIM as a novel tumor suppressor in MCL.

### [Example 2]

### (Relationship between genomic aberrations and prognosis)

With respect to MCL specimens in 29 cases as in Example 1, relationship with prognosis was examined. Fig. 6 shows prognosis curves for gain or loss of specific clones. That is, with respect to 29 cases, the relationship between specific gains or losses and prognosis (survival state) was examined using Kaplan-Meier survival curves and the log-rank test. Fig. 6 shows Kaplan-Meier survival curves. The abscissa represents the number of days of survival, and the ordinate represents the survival probability. As shown in Fig. 6, cases with 6p21 (BAC, RP11-60O19) loss (6 cases) had a significantly better prognosis (P = 0.0152) than those without loss (23 cases). Furthermore, cases with 8p23 (BAC, RP11-240A17) loss (9 cases) had a significantly poorer prognosis (P = 0.0001) than those without loss (20 cases), and cases with 8q24 (RP11-1136L8) gain (5 cases) had a significantly poorer prognosis (P = 0.0084) than those without gain (24 cases). Although the causative genes on 6q21 and 8p22 are unknown, according to this example, the causative genes on 8q24 is strongly suggested to be C-MYC. Additionally, no significant differences were shown with respect to the relationship between prognosis and genomic aberrations in the regions in which deletions occur with high frequency, such as 1p22, 9p21, 11q21, and 13q21.

The entire contents of the folowing references are incorporated herein by reference.
Allen JE, Hough RE, Goepel JR, Bottomley S, Wilson GA, Alcock HE, Baird M, Lorigan PC, Vandenberghe EA, Hancock BW and Hammond DW. (2002). Br J Haematol., 116, 291-298.
Amin HM, McDonnell TJ, Medeiros LJ, Rassidakis GZ, Leventaki V, O'Connor SL, Keating MJ and Lai R. (2003). Arch Pathol Lab Med., 127, 424-431. Beà S, Ribas M, Hernández JM, Bosch F, Pinyol M, Hernández L, Garcia JL, Flores T, González M, Lopez-Guillermo A, Piris MA, Cardesa A, Montserrat E , Miró R and Campo E. (1999). Blood., 93, 4365-4374.
Beà S, Tort F, Pinyol M, Puig X, Hernández L, Hernández S, Fernández PL, Lohuizen M, Colomer D and Campo E. (2001). Cancer Res., 61, 2409-2412.
Bentz M, Plesch A, Bullinger L, Stilgenbauer S, Ott G, Muller-Hermelink HK, Baudis M, Barth TF, Moller P, Lichter P and Dohner H. (2000). Genes Chromosomes Cancer., 27, 285-294.
Bigoni R, Cuneo A, Milani R, Roberti MG, Bardi A, Rigolin GM, Cavazzini F, Agostini P and Castoldi G. (2001). Leuk Lymphoma., 40: 581-590.
Bouillet P, Purton JF, Godfrey DI, Zhang LC, Coultas L, Puthalakath H, Pellegrini M, Cory S, Adams JM and Strasser A. (2002). Nature., 415, 922-926.
Cogen PH, Daneshvar L, Metzger AK, Duyk G, Edwards MS and Sheffield VC. (1992). Am J Hum Genet., 50, 584-589.
Cuneo A, Bigoni R, Rigolin GM, Roberti MG, Bardi A, Piva N, Milani R, Bullrich F, Veronese ML, Croce C, Birg F, Dohner H, Hagemeijer A and Castoldi G. (1999). Blood., 93, 1372-1380.
Dreyling MH, Bullinger L, Ott G, Stilgenbauer S, Muller-Hermelink HK, Bentz M, Hiddemann W and Dohner H. (1997). Cancer Res., 57, 4608-4614.
Egle A, Harris AW, Bouillet P and Cory S. (2004). Proc Natl Acad Sci U S A., 101, 6164-6169.
Enders A, Bouillet P, Puthalakath H, Xu Y, David M. Tarlinton DM and Strasser A. (2003). J Exp Med., 198, 1119-1126.
Fujimori M, Tokino T, Hino O, Kitagawa T, Imamura T, Okamoto E, Mitsunobu M, Ishikawa T, Nakagama H, Harada H, Yagura M, Matsubara K and Nakamura Y. (1991). Cancer Res., 51, 89-93.
Greiner TC, Moynihan MJ, Chan WC, Lytle DM, Pedersen A, Anderson JR and Weisenburger DD. (1996). Blood., 87, 4302-4310.
Hernandez L, Fest T, Cazorla M, Teruya-Feldstein J, Bosch F, Peinado MA, Piris MA, Montserrat E, Cardesa A, Jaffe ES, Campo E and Raffold M. (1996). Blood., 87, 3351-3359.
Hinds PW, Dowdy SF, Eaton EN, Arnold A and Weinberg RA. (1994). Proc Natl Acad Sci U S A., 91, 709.
Hofmann W-K, de Vos S, Tsukasaki K, Wachsman W, Pinkus GS, Said JW and Koeffler P. (2001). Blood., 98, 787-794.
Jaffe ES. (ed). (2001). World health classification of tumors: Pathology & Genetics of tumours of haematopoietic and lymphoid tissues. Washington: IARC press, Lyon. Jeon HJ, Kim CW, Yoshino T and Akagi T. (1998). Br J Haematol., 102, 1323-1326.
Knudson AG Jr. (1971). Proc Natl Acad Sci U S A., 68, 820-823. Kohlhammer H, Schwaenen C, Wessendorf S, Holzmann K, Kestler HA, Kienle D, Barth T, Moller P, Ott G, Kalla J, Radlwimmer B, Pscherer A, Stilgenbauer S, Dohner H, Lichter P and Bentz M. (2004). Blood., 104, 795-801.
Konishi H, Nakagawa T, Harano T, Mizuno K, Saito H, Masuda A, Matsuda H, Osada H and Takahashi T. (2002). Cancer Res., 62, 271-276.
Liu J-W, Chandra D, Tang S-H, Chopra D and Tang DG. (2002). Cancer Res., 62, 2976-2981.
Lovec H, Grzeschiczek A, Kowalski B and Moroy T. (1994). EMBO J., 13, 3487-3495.
Martinez-Climent JA, Vizcarra E, Sanchez D, Blesa D, Rubio-Moscardo F, Albertson DG, Garcia-Conde J, Dyer M.J.S, Levy R, Pinkel D and Lossos IS. (2001). Blood., 98, 3479-3482.
Martinez N, Camacho FI, Algara P, Rodriguez A, Dopazo A, Ruiz-Ballesteros E, Martin P, Martinez-Climent JA, Garcia-Conde J, Menárguez J, Solano F, Mollejo M and Piris MA. (2003). Cancer Res., 63, 8226-832.
Monni O, Oinonen R, Elonen E, Franssila K, Teerenhovi L, Joensuu H and Knuutila S. (1998). Genes Chromosomes Cancer., 21, 298-307. Motegi M, Yonezumi M, Suzuki H, Suzuki R, Hosokawa Y, Hosaka S, Kodera Y, Morishima Y, Nakamura S and Seto M. (2000). Am J Pathol., 156, 807-812.
Nacheva E, Dyer MJ, Fischer P, Stranks G, Heward JM, Marcus RE, Grace C and Karpas A. (1993). Blood., 82, 231-240.
Nomura K, Yoshino T, Nakamura S, Akano Y, Tagawa H, Nishida K, Seto M, Nakamura S, Ueda R, Yamagishi H and Taniwaki M. (2003). Cancer Genet Cytogenet., 140, 49-54.
Ota A, Tagawa H, Karnan S, Karpas A, Kira S, Yoshida Y and Seto M. (2004). Cancer Res., 64, 3087-3095.
O'Connor L, Strasser A, O'Reilly LA, Hausmann G, Adams JM, Cory S and Huang DC. (1998). EMBO J., 17, 384-395.
Phillips NJ, Ziegler MR, Radford DM, Fair KL, Steinbrueck T, Xynos FP and Donis-Keller H. (1996). Cancer Res., 56, 606-611.
Pinkel D, Segraves R, Sudar D, Clark S, Poole I, Kowbel D, Collins C, Kuo WL, Chen C, Zhai Y, Dairkee SH, Ljung BM and Gray JW, Albertson DG. (1998). Nat Genet., 1998, 23: 41-46.
Pinyol M, Hernandez L, Cazorla M, Balbín M, Jares P, Fernandez PL, Montserrat E, Cardesa A, Lopez-Otin C and Campo E. (1997). Blood., 89, 272-280.
Rosenwald A, Wright G, Wiestner A, Chan WC, Connors JM, Campo E, Gascoyne RD, Grogan TM, Muller-Hermelink HK, Smeland EB, Chiorazzi M, Giltnane JM, Hurt EM, Zhao H, Averett L, Henrickson S, Yang L, Powell J, Wilson WH, Jaffe ES, Simon R, Klausner RD, Montserrat E, Bosch F, Greiner TC, Weisenburger DD, Sanger WG, Dave BJ, Lynch JC, Vose J, Armitage JO, Fisher RI, Miller TP, LeBlanc M, Ott G, Kvaloy S, Holte H, Delabie J and Staudt LM. (2003). Cancer Cell, 3, 185-197.
Saltman DL, Cachia PG, Dewar AE, Ross FM, Krajewski AS, Ludlam C and Steel CM. (1998). Blood., 72, 2026-2030.
Saxena A, Clark WC, Robertson JT, Ikejiri B, Oldfield EH and Ali IU. (1992). Cancer Res., 52, 6716-6721.
Schaffner C, Idler I, Stilgenbauer S, Dohner H and Lichter P. (2000). Proc Natl Acad Sci U S A., 97, 2773-2778.
Schultz DC, Vanderveer L, Berman DB, Hamilton TC, Wong AJ and Godwin AK. (1996). Cancer Res., 56, 1997-2002.
Seto M, Yamamoto K, Iida S, Akao Y, Utsumi KR, Kubonishi I, Miyoshi I, Ohtsuki T, Yawata Y, Namba and Ueda R. (1992). Oncogene., 7, 1401-1406.
Stilgenbauer S, Winkler D, Ott G, Schaffner C, Leupolt E, Bentz M, Moller P, Muller-Hermelink HK, James MR, Lichter P and Dohner H. (1999). Blood., 94, 3262-3264.
Suzuki R, Kuroda H, Komatsu H, Hosokawa Y, Kagami Y, Ogura M, Nakamura S, Kodera Y, Morishima Y, Ueda R and Seto M. (1999). Leukemia., 13, 1335-1342.
U M, Miyashita T, Shikama Y, Tadokoro K and Yamada M. (2001). FEBS Lett., 509, 135-141.
Zhang X. Karnan S, Tagawa H, Suzuki R, Morishima Y, Nakamura S and Masao Seto (2004). Cancer Science in press.

The present invention claims priority from U.S Provisional Application No. 60/632,708 filed on December 3, 2004 and U.S. Provisional Application No. 60/722,007 filed on September 30, 2005, the entire contents of both of which are incorporated herein by reference.

### Industrial Applicability

The present invention can be used for diagnosis and Description of Sequence

Sequence ID Nos. 3 to 8 Probes for detecting deletion in human chromosome 2 q13
Sequence ID No. 9 Sense primer for probe 1
Sequence ID No. 10 Antisense primer for probe 1
Sequence ID No. 11 Sense primer for probe 2
Sequence ID No. 12 Antisense primer for probe 2
Sequence ID No. 13 Sense primer for probe 3
Sequence ID No. 14 Antisense primer for probe 3
Sequence ID No. 15 Sense primer for probe 4
Sequence ID No. 16 Antisense primer for probe 4
Sequence ID No. 17 Sense primer for probe 5
Sequence ID No. 18 Antisense primer for probe 5
Sequence ID No. 19 Sense primer for probe 6
Sequence ID No. 20 Antisense primer for probe 6
Sequence ID No. 21 Sense primer for probe 7
Sequence ID No. 22 Antisense primer for probe 7
Sequence ID No. 23 Sense primer for probe 8
Sequence ID No. 24 Antisense primer for probe 8
Sequence ID No. 25 Sense primer for RT-PCR
Sequence ID No. 26 Antisense primer for RT-PCR

## Claims

1. A method for diagnosis of a malignant tumor comprising a step of detecting a deletion or mutation in one or two or more selected from the group consisting of human chromosome 1 p36.23 to p36.32, human chromosome 1 q42.2 to q43, human chromosome 2 p11.2, human chromosome 2 q13, human chromosome 17 p11.2 to p13.3, and human chromosome 19 p13.2 to p13.3.

2. The method for diagnosis according to Claim 1, wherein the malignant tumor is a malignant lymphoma.

3. The method for diagnosis according to Claim 1 or 2, further comprising a disease type determination step of determining the type of the malignant lymphoma on the basis of the detection result in the detection step.

4. The method for diagnosis according to Claim 3, wherein the disease type is determined to be mantle cell lymphoma.

5. The method for diagnosis of a malignant tumor according to any one of Claims 1 to 4, further comprising a step of detecting a deletion or mutation in the base sequence of human chromosome 2 q13.

6. The method for diagnosis according to Claim 5, wherein the detection step is a step of detecting a deletion or mutation of the BIM gene.

7. The method for diagnosis according to Claim 5 or 6, further comprising a therapeutic response determination step of determining a therapeutic response of the malignant tumor on the basis of the detection result in the detection step.

8. The method for diagnosis according to any one of Claims 5 to 7, wherein the detection step is a step of detecting a deletion or mutation in any of BAC RP11-438K19 35027bp to 49920bp, the 394th base to the 597th base in the base sequence described in Sequence ID No. 1, and the 214th base to the 417th base in the base sequence described in Sequence ID No. 2.

9. The method for diagnosis according to any one of Claims 5 to 8, wherein the detection step is a step of using any of the BIM gene, and mRNA and cDNA expressed from the BIM gene, in a specimen.

10. The method for diagnosis according to Claim 9, wherein the detection step includes carrying out PCR, RT-PCR, or nucleic acid hybridization.

11. The method for diagnosis according to Claim 9, wherein the detection step includes hybridizing, on an array provided with one or two or more probes complementary to at least a part of the BIM gene, the probes to a nucleic acid sample prepared from the specimen.

12. The method for diagnosis according to any one of Claims 1 to 11, wherein the detection step is a step of performing array CGH.

13. The method for diagnosis according to any one of Claims 5 to 8, wherein the detection step is a step of detecting the presence or absence of expression, an expression level, or a mutation of a protein encoded by the BIM gene.

14. A diagnostic marker for malignant tumors, wherein the diagnostic marker is the BIM gene, a part of the BIM gene, or a polynucleotide having a base sequence complementary thereto.

15. The diagnostic marker according to Claim 14, wherein a protein translation region of the BIM gene has a base sequence described in Sequence ID No. 1 or in Sequence ID No. 2.

16. The diagnostic marker according to Claim 14, wherein the diagnostic marker is at least a part of the 394th base to the 597th base in the base sequence described in Sequence ID No. 1 or the 214th base to the 417th base in the base sequence described in Sequence ID No. 2, or a polynucleotide having a base sequence complementary thereto.

17. The diagnostic marker according to any one of Claims 14 to 16, wherein the marker is used as a probe or a primer.

18. A diagnostic marker for malignant tumors, wherein the diagnostic marker is a protein encoded by the BIM gene, a part thereof, or an antibody thereto.

19. An array for diagnosis of malignant tumors, in which a nucleic acid probe for detecting a deletion or mutation in human chromosome 2 q13 is immobilized.

20. The array for diagnosis according to Claim 19, wherein the nucleic acid probe is a nucleic acid probe for detecting a deletion or mutation of the BIM gene.

21. The array for diagnosis according to Claim 19, wherein the nucleic acid probe has at least a part of the 394th base to the 597th base in the base sequence described in Sequence ID No. 1 or the 214th base to the 417th base in the base sequence described in Sequence ID No. 2, or a base sequence complementary thereto.

22. A diagnostic kit for the method for diagnosis according to any one of Claims 1 to 11, comprising a nucleic acid probe for detecting a deletion or mutation of the BIM gene.

23. A pharmaceutical composition for treating mantle cell lymphoma, containing a DNA construct including a coding region of the BIM gene or a coding region of a homologuous protein having an activity of a protein encoded by the BIM gene.

24. A pharmaceutical composition for treating mantle cell lymphoma, containing a protein encoded by the BIM gene or a homologuous protein having an activity of a protein encoded by the BIM gene.

25. A method for prognosis of a mantle cell lymphoma patient, the method comprising a step of detecting a deletion or mutation in human chromosome 6 q16.2 to q27, a deletion or mutation in human chromosome 8 p12 to p23.2, and an amplification or mutation in human chromosome 8 q13.2 to q24.22 with respect to a sample collected from the patient.

26. The method according to Claim 25, wherein any of the determination steps (a) to (c) described below is carried out:
(a) when a deletion is detected in human chromosome 6 q16.2 to q27, the prognosis is good;
(b) when a deletion is detected in human chromosome 8 p12 to p23.2, the prognosis is poor; and
(c) when an amplification is detected in human chromosome 8 q13.2 to q24.22, the prognosis is poor.

27. The method according to Claim 25 or 26, wherein the detection step includes detecting the deletion or amplification by hybridization of a probe containing a region on the chromosome to a nucleic acid sample collected from the patient.

28. The method according to Claim 27, wherein the probe is a BAC clone and/or a PAC clone.

29. The method according to Claim 28, wherein any one of a BAC clone (RP11-60019), a BAC clone (RP11-240A17), and a BAC clone (RP11-1136L8) or a PAC clone (RP1-80K22) is used.

30. The method according to Claim 25 or 26, wherein the detection step is a step of detecting an amplification or mutation of the c-MYC gene.

31. The method according to Claim 25 or 26, wherein the detection step is a step of detecting the presence or absence, an expression level, or a mutation of a protein encoded by the c-MYC gene.

32. The method according to any one of Claims 25 to 31, wherein the detection step is carried out on a solid-phase carrier.

33. An array for prognosis of mantle cell lymphoma, in which any of a probe for detecting human chromosome 6 q16.2 to q27, a probe for detecting human chromosome 8 p12 to p23.2, and a probe for detecting human chromosome 8 q13.2 to q24.22 is immobilized.

34. A marker for prognosis of mantle cell lymphoma, the marker being a polynucleotide for detecting human chromosome 6 q16.2 to q27, human chromosome 8 p12 to p23.2, and human chromosome 8 q13.2 to q24.22.

35. The marker according to Claim 34, wherein the polynucleotide is the c-MYC gene, a part of the c-MYC gene, or a polynucleotide having a base sequence complementary thereto.

36. A marker for prognosis of mantle cell lymphoma, the marker being a protein encoded by the c-MYC gene, a part of the protein, or an antibody thereto.
